# EUROPEAN PATENT SPECIFICATION

(11) **EP 3 924 733 B1**
(45) Date of publication and mention of the grant of the patent: **28.08.2024**
(21) Application number: 20710020.7
(22) Date of filing: 13.02.2020
(51) Int. Cl.: G01N 33/50, G01N 33/574

(54) **CCR5-BASED METHODS FOR PREDICTING OVERALL AND PROGRESSION FREE SURVIVAL IN SUBJECTS HAVING CANCER**
CCR5-BASIERTE VERFAHREN ZUR VORHERSAGE DES GESAMTÜBERLEBENS UND DES PROGRESSIONSFREIEN ÜBERLEBENS BEI KREBSKRANKEN
PROCÉDÉS À BASE DE CCR5 POUR PRÉDIRE LA SURVIE GLOBALE ET SANS PROGRESSION CHEZ DES SUJETS ATTEINTS D'UN CANCER

(30) Priority: 13.02.2019 US 201962805031 P
(43) Date of publication of application: 22.12.2021
(73) Proprietor: Creatv Microtech, Inc., Potomac, MD 20854 (US)
(72) Inventor: ADAMS, Daniel L., Basking Ridge, New Jersey 07920 (US); TANG, Cha-Mei, Potomac, Maryland 20854 (US)
(74) Representative: Dehns
(86) International application number: PCT/US2020/018074
(87) International publication number: WO 2020/168052

(56) References cited:
- WO-A1-2016/209926
- WO-A1-2018/213732
- SINGER IRWIN I ET AL: "CCR5, CXCR4, and CD4 are clustered and closely apposed on microvilli of human macrophages and T cells", JOURNAL OF VIROLOGY, vol. 75, no. 8, 15 April 2001 (2001-04-15), pages 3779 - 3790, DOI: doi.org/10.1128/jvi.75.8.3779-3790.2001
- RAGHAVAKAIMAL ASHVATHI ET AL: "CCR5 activation and endocytosis in circulating tumor-derived cells isolated from the blood of breast cancer patients provide information about clinical outcome.", BREAST CANCER RESEARCH, vol. 24, 35, 23 May 2022 (2022-05-23), pages 1 - 12, DOI: 10.1186/s13058-022-01528-w
- YOSHIKANE YAMAUCHI ET AL: "Circulating and Tumor Myeloid-derived Suppressor Cells in Resectable Non-Small Cell Lung Cancer", AMERICAN JOURNAL OF RESPIRATORY AND CRITICAL CARE MEDICINE., vol. 198, no. 6, 15 September 2018 (2018-09-15), US, pages 777 - 787, XP055696819, ISSN: 1073-449X, DOI: 10.1164/rccm.201708-1707OC
- D. L. ADAMS ET AL: "Circulating Cancer-Associated Macrophage-Like Cells Differentiate Malignant Breast Cancer and Benign Breast Conditions", CANCER EPIDEMIOLOGY, BIOMARKERS AND PREVENTION., vol. 25, no. 7, 17 May 2016 (2016-05-17), US, pages 1037 - 1042, XP055479663, ISSN: 1055-9965, DOI: 10.1158/1055-9965.EPI-15-1221

## Description

### BACKGROUND

### Field of the Invention

The present invention generally relates to the use of biomarkers in the blood and other bodily fluids to provide companion and complementary diagnostics and to make predictions regarding overall survival and progression free survival in subjects having cancer, such as solid tumors.

### Related Art

When tumor cells break away from primary solid tumors, they penetrate into the blood or lymphatic circulation, and ultimately leave the blood stream and enter either organs or tissue to form metastasis. 90% of cancer-related deaths are caused by the metastatic process. The most common metastatic sites are the lung, liver, bone and brain. Tumor cells found in the circulation are called circulating tumor cells (CTCs). Many research publications and clinical trials show that CTCs have clinical utility in (i) providing prognostic survival and cancer recurrence information through the enumeration of CTCs in the blood stream, and (ii) providing treatment information through examination of protein expression levels, and the occurrence of gene mutations and translocations in the CTCs. However, CTCs are not consistently associated with the development and/or presence of cancer in a subject, even in stage IV cancer patients. While CTCs are found most often in stage IV of breast, prostate and colorectal cancers, they are rare in early stages of the same cancer. CTCs are also rare in other cancers.

Circulating cancer associated macrophage-like cells (CAMLs) are another cancer-related cell type that is found in the blood of subjects having cancer. CAMLs are associated with all solid tumors tested and all stages of cancer. CAMLs are polyploid and very large in size, ~25 µm to ~300 µm in size. These polyploid cells can be either CD45(-) or CD45(+) and express CD11c, CD 14 and CD31, which confirms their origin as a myeloid lineage. They are often found in the process of engulfing CTCs and cell debris ^{[14,22]}. The examination of protein expression levels in CAMLs can also assist clinicians in making informed treatment decisions.

Assays associated with the identification and characterization of biomarkers in CTCs and CAMLs, in blood and other body fluids, can be used to provide important prognostic and treatment information for a subject having cancer. The present invention is directed to providing such tools to clinicians and other important goals.

WO 2016/209926 discloses methods for treating cancer using CCR5 inhibitors and detecting CCR5 on circulating tumor cells.

WO 2018/213732 discloses methods of treatment for cancer(s) through combination therapy with an agent that inhibits poly [ADP-ribose] polymerase (PARP) signaling and an agent that regulates activity within the tumor microenvironment.

Yamauchi Y, et al. Am J Respir Crit Care Med. 2018;198(6):777-787, discusses circulating and tumor myeloid-derived suppressor cells in resectable non-small cell lung cancer.

Adams D.L., et al. Cancer Epidemiol Biomarkers Prev. 2016;25(7):1037-1042, discloses that circulating cancer-associated macrophage-like cells differentiate malignant breast cancer and benign breast conditions.

### SUMMARY

The present invention is related to methods for predicting overall survival (OS) and progression free survival (PFS) of a subject having cancer, based on the relative amount of the protein receptor CCR5 found in circulating cells of the subject. While the circulating cells can be one or more of several different cell types (defined below), at a minimum CCR5 expression is determined in a type of cell with unique characteristics that is found in the blood of subjects having solid tumors, including carcinoma, sarcoma, neuroblastoma and melanoma. These circulating cells, termed a "circulating Cancer Associated Macrophage-like cell" (CAML), have been shown to be associated with the presence of solid tumors in a subject having cancer. Five morphologies associated with CAMLs have been characterized and described ^{[14,21]}. CAMLs have been found consistently in the peripheral blood of subjects having stage I to stage IV solid tumors by microfiltration using precision microfilters.

Medical applications associated with CAMLs include, but are not limited to, use of the cells themselves as a biomarker to provide early detection of cancer and diagnosis of cancer, in particular, in the early detection and diagnosis of cancer relapse or recurrence, and in the determination of cancer mutation. CAMLs are also shown through the data presented herein as expressing biomarkers having clinical utility in making predictions regarding disease progression and patient survival, such as CCR5, which can aid in making informed treatment decisions.

Biomarker expression by CAMLs may be used independently as a cancer marker, or in combination with biomarker expression by other circulating cells, such as circulating tumor cells (CTCs), CTC subtypes undergoing epithelial mesenchymal transition cells (EMTCTCs), circulating cancer associated vascular endothelial cells (CAVEs), as well as the presence of these additional cell types themselves and also cell-free DNA (cfDNA), circulating tumor DNA (ctDNA), methylated DNA, proteomic, metabolomic, lipidomic and other biomarkers to provide a more complete understanding of a patient's disease.

Embodiments of the present invention are defined in the claims. In particular, in a first embodiment, the present invention is directed to a method for predicting overall survival (OS) and progression free survival (PFS) of a subject having cancer, said method comprising determining thenumber of CCR5 clusters in circulating cells in a biological sample from a subject having cancer, wherein the circulating cells comprise cancer associated macrophage-like cells (CAMLs), and wherein the CAMLs have the following characteristics: (a) large atypical polyploid nucleus of about 14-64 µm in size, or multiple nuclei in a single cell; (b) cell size of about 20-300 µm in size; and (c) morphological shape selected from the group consisting of spindle, tadpole, round, oblong, two legs, more than two legs, thin legs, and amorphous; optionally wherein the CAMLs have one or more of the following additional characteristics: (d) CD14 positive phenotype; (e) CD45 expression; (f) EpCAM expression; (g) vimentin expression; (h) PD-L1 expression; (i) CD11C marker expression; (j) CD146 marker expression; (k) CD202b marker expression; (l) CD31 marker expression; and (m) CK8, 18, 19 epithelial phenotype, wherein the source of the biological sample is one or more of peripheral blood, blood, lymph node, bone marrow, cerebral spinal fluid, tissue, and urine, and wherein when at least one CAML cell in said sample has about 10 or more CCR5 clusters: (i) the OS and PFS of the subject is predicted to be shortened, or (ii) the OS and PFS of the subject is predicted to be shorter than a subject having the same type of cancer where none of the cells in a corresponding biological sample has about 10 or more CCR5 clusters.

In some aspects of this embodiment, the method comprises determining the number of CCR5 pools/clusters in circulating cells in a biological sample from a subject having cancer, wherein when at least one cell in said sample has about 15 or more CCR5 pools/clusters, the OS and PFS of the subject is predicted to be shortened; or
wherein when at least one cell in said sample has about 16 or more CCR5 pools/clusters, the OS and PFS of the subject is predicted to be shortened; or
wherein when at least one cell in said sample has about 17 or more CCR5 pools/clusters, the OS and PFS of the subject is predicted to be shortened; or
wherein when at least one cell in said sample has about 18 or more CCR5 pools/clusters, the OS and PFS of the subject is predicted to be shortened; or
wherein when at least one cell in said sample has about 19 or more CCR5 pools/clusters, the OS and PFS of the subject is predicted to be shortened; or
wherein when at least one cell in said sample has about 20 or more CCR5 pools/clusters, the OS and PFS of the subject is predicted to be shortened; or
wherein when at least one cell in said sample has about 21 or more CCR5 pools/clusters, the OS and PFS of the subject is predicted to be shortened; or
wherein when at least one cell in said sample has about 22 or more CCR5 pools/clusters, the OS an PFS of the subject is predicted to be shortened; or
wherein when at least one cell in said sample has about 23 or more CCR5 pools/clusters, the OS and PFS of the subject is predicted to be shortened; or
wherein when at least one cell in said sample has about 24 or more CCR5 pools/clusters, the OS and PFS of the subject is predicted to be shortened; or
wherein when at least one cell in said sample has about 25 or more CCR5 pools/clusters, the OS and PFS of the subject is predicted to be shortened.

In other aspects of this embodiment, the method comprises determining the number of CCR5 pools/clusters in circulating cells in a biological sample from a subject having cancer, wherein when at least one cell in said sample has about 15 or more CCR5 pools/clusters, the OS and PFS of the subject is predicted to be shorter than a subject having the same type of cancer where none of the cells in a corresponding biological sample has about 15 or more CCR5 pools/clusters; or
wherein when at least one cell in said sample has about 16 or more CCR5 pools/clusters, the OS and PFS of the subject is predicted to be shorter than a subject having the same type of cancer where none of the cells in a corresponding biological sample has about 16 or more CCR5 pools/clusters; or
wherein when at least one cell in said sample has about 17 or more CCR5 pools/clusters, the OS and PFS of the subject is predicted to be shorter than a subject having the same type of cancer where none of the cells in a corresponding biological sample has about 17 or more CCR5 pools/clusters; or
wherein when at least one cell in said sample has about 18 or more CCR5 pools/clusters, the OS and PFS of the subject is predicted to be shorter than a subject having the same type of cancer where none of the cells in a corresponding biological sample has about 18 or more CCR5 pools/clusters; or
wherein when at least one cell in said sample has about 19 or more CCR5 pools/clusters, the OS and PFS of the subject is predicted to be shorter than a subject having the same type of cancer where none of the cells in a corresponding biological sample has about 19 or more CCR5 pools/clusters; or
wherein when at least one cell in said sample has about 20 or more CCR5 pools/clusters, the OS and PFS of the subject is predicted to be shorter than a subject having the same type of cancer where none of the cells in a corresponding biological sample has about 20 or more CCR5 pools/clusters; or
wherein when at least one cell in said sample has about 21 or more CCR5 pools/clusters, the OS and PFS of the subject is predicted to be shorter than a subject having the same type of cancer where none of the cells in a corresponding biological sample has about 21 or more CCR5 pools/clusters; or
wherein when at least one cell in said sample has about 22 or more CCR5 pools/clusters, the OS and PFS of the subject is predicted to be shorter than a subject having the same type of cancer where none of the cells in a corresponding biological sample has about 22 or more CCR5 pools/clusters; or
wherein when at least one cell in said sample has about 23 or more CCR5 pools/clusters, the OS and PFS of the subject is predicted to be shorter than a subject having the same type of cancer where none of the cells in a corresponding biological sample has about 23 or more CCR5 pools/clusters; or
wherein when at least one cell in said sample has about 24 or more CCR5 pools/clusters, the OS and PFS of the subject is predicted to be shorter than a subject having the same type of cancer where none of the cells in a corresponding biological sample has about 24 or more CCR5 pools/clusters; or
wherein when at least one cell in said sample has about 25 or more CCR5 pools/clusters, the OS and PFS of the subject is predicted to be shorter than a subject having the same type of cancer where none of the cells in a corresponding biological sample has about 25 or more CCR5 pools/clusters.

In a second embodiment, the present invention is directed to a method for predicting OS and PFS of a subject having cancer, said method comprising (i) determining the number of CCR5 clusters in circulating cells in a biological sample from a first subject having cancer, and comparing the results to those determined from a second subject having the same type of cancer, wherein the OS and PFS of the subject having a greater number of CCR5 clusters is predicted to be shorter than the OS and PFS of the subject having a fewer CCR5 clusters; (ii) determining the mean number of CCR5 clusters in a selected number of circulating cells in a biological sample from a first subject having cancer, and comparing the results with those determined from a second subject having the same type of cancer, wherein the OS and PFS of the subject with a greater mean number of CCR5 clusters is predicted to be shorter than the OS and PFS of the subject having a lesser mean number of CCR5 clusters; or (iii) determining the median number of CCR5 clusters in a selected number of circulating cells in a biological sample from a first subject having cancer, and comparing the results with those determined from a second subject having the same type of cancer, wherein the OS and PFS of the subject with a greater median number of CCR5 clusters is predicted to be shorter than the OS and PFS of the subject having a lesser median number of CCR5 clusters, wherein the circulating cells comprise cancer associated macrophage-like cells (CAMLs) and wherein the CAMLs have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous,
and wherein the source of the biological sample is one or more of peripheral blood, blood, lymph node, bone marrow, cerebral spinal fluid, tissue, and urine.

In certain aspects of the embodiments of the invention, the CAMLs can be further identified as having one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype.

In each of the embodiments and aspects of the invention, the CCR5 clusters/pools may be localized to the nucleus of the cell, or localized to the cytoplasm of the cell, or localized to the surface of the cell, or some combination thereof.

In each of the embodiments and aspects of the invention, the CCR5 of the circulating cells may be in an activated state, or an inactivated state, or the cells may have both activated and inactivated receptors.

In certain aspects of the embodiments of the invention, OS or PFS, or both, is over a period of at least 12 months. In other aspects of the embodiments of the invention, OS or PFS, or both, is over a period of at least 24 months.

In certain aspects of the embodiments of the invention, the size of the biological sample is between 5 and 15 mL.

In each aspect and embodiment of the invention, the circulating cells further comprise one or more of CTCs, PDCTCs, apoptotic CTCs, EMTCTCs and CAVEs. Thus, the methods of the invention can be performed by assaying CCR5 expression in CAMLs alone, or the methods of the invention can be performed by assaying CCR5 expression in CAMLs and one or more of CTCs, PDCTCs, apoptotic CTCs, EMTCTCs and CAVEs.

In certain aspects of the embodiments of the invention, when the biological sample is blood, the blood may be antecubital-vein blood, inferior-vena-cava blood, femoral vein blood, portal vein blood, or jugular-vein blood. The sample may be a fresh sample or a properly prepared cryo-preserved sample that is thawed.

In each aspect and embodiment of the invention, the cancer is one where the cells thereof express CCR5. The CCR5-expressing cancers may be a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

In certain aspects of the embodiments of the invention, the circulating cells are isolated from the biological samples for the determining steps using one or more means selected from size exclusion methodology, immunocapture, red blood cell lysis, white blood cell depletion, FICOLL, electrophoresis, dielectrophoresis, flow cytometry, magnetic levitation, and various microfluidic chips, or a combination thereof.

In one aspect of the invention, circulating cells are isolated from the biological samples using size exclusion methodology that comprises using a microfilter. The microfilter may have a pore size ranging from about 5 microns to about 20 microns. The pores of the microfilter may have any shape, with acceptable shapes including round, race-track shape, oval, slit, square, rectangular and/or other shapes. The microfilter may have precision pore geometry, uniform pore distribution, more than one pore geometry, and/or non-uniform pore distribution. The microfilter may be single layer, or multi-layers with different shapes on different layers.

In another aspect of the invention, circulating cells are isolated from the biological samples using a microfluidic chip via physical size-based sorting, slits, channels, hydrodynamic size-based sorting, grouping, trapping, immunocapture, concentrating large cells, eliminating small cells based on size, or a combination thereof.

In a further aspect of the invention, circulating cells are isolated from the biological samples using a low-pressure microfiltration assay.

### BRIEF DESCRIPTION OF THE DRAWINGS

**Figure** 1. Images of a CAML stained for CCR5.
**Figure 2****.** Images of a CAMI, stained for CCR5.
**Figures 3A-3C****.** Images of CAMLs stained for CCR5. Medium/high levels of CCR5 expression are seen in the cytoplasm; the receptor is inactivated and not translocated to the nucleus.
**Figures 4A-4C****.** Images of CAMLs stained for CCR5. Low/negative levels of CCR5 expressions are seen in the cytoplasm; the receptor is inactivated and not translocated to the nucleus.
**Figures 5A-5C****.** Images of CAMLs stained for CCR5. High levels of CCR5 expression is seen in these CAMLs. CCR5 pools/clusters in Figures 5B-5C are mostly in the nucleus; the receptor is inactivated and translocated to the nucleus.
**Figures 6A-6B****.** Images of CAMLs stained for CCR5. Medium/low levels of CCR5 expression is seen in the nucleus; the CCR5 pools/clusters in Figure 6B are mostly inactivated and translocated to the nucleus.
**Figure** 7. Images of EMTCTCs stained for CCR5 showing CCR5 expression aggregated in one location on the surface of the cell.
**Figure 8****.** Images of EMTCTCs stained for CCR5 showing no CCR5 expression.
**Figures 9A-9C****.** Images in these panels demonstrate the variability in CCR5 straining that is seen in some cells. CTCs are stained for CCR5 and show moderate levels of CCR5 expression in Figs. 9A and 9B, and very high levels of CCR5 expression in Fig. 9C.
**Figures 10A-10B****.** Images of CTCs stained for CCR5 showing low CCR5 expression.
**Figure 11****.** Kaplan-Meier plots showing overall survival and progression free survival based on the number of CCR5 pools/clusters in CAMLs.
**Figure 12****.** The intensity of cell differentiation markers used to identify and subtype CAMLs is shown.

### DETAILED DESCRIPTION

Liquid biopsies provide real-time, sequential tracking of diagnostically-important circulating cells isolated from a subject having cancer. Cells such as circulating tumor cells (CTCs) are found in the peripheral blood of cancer patients and previous work has shown that CTC-based assays can be used as a substitute to tissue biopsies ^{[1-4]}. Different subgroups of CTCs upregulate and/or down regulate phenotypes and marker expression in relation to tumor progression, tumor spread, and in response to tumor treatments. Therefore, assessing CTCs and the markers expressed by such cells in the peripheral blood can provide important information regarding the status of the cancer in the subject.

CTCs that can be used as cancer diagnostics, as well as means for screening and monitoring treatment and determining the susceptibility of a tumor in a particular subject to a particular treatment, can be divided into three subgroups. The first subgroup is pathologically definable CTCs (PDCTCs) ^{[6-10]}. In PDCTCs, the cytokeratin pattern is filamentous. The second subgroup is apoptotic CTCs. When a CTC dies, the cytokeratin pattern degrades into dots. Therefore, early apoptotic CTC have some cytokeratin dots and later apoptotic CTCs have all of the cellular cytokeratin degraded into dots. In the third subgroup of CTCs, the cells are undergoing epithelial to mesenchymal transition (EMTCTCs) ^{[2, 3, 5-9]}. EMT is a gradual morphogenetic process, and EMTCTCs encompass cells in various stages of transition ^{[6]}. EMTCTCs can be generally described by the down regulation of epithelial proteins, e.g. EpCAM and CK, and the upregulation of mesenchymal stem cell proteins, e.g. vimentin and CD34 ^{[13]}. EMTCTC subtyping is typically performed using non-proteomic methods, i.e. mRNA expression or DNA analysis ^{[13]}.

A further type of circulating cell associated with cancer that may serve as a diagnostic is the cancer-associated vascular endothelial cell or CAVE. CAVEs are a subtype of circulating endothelial cells. Tumors require blood supply provided by tumor endothelial cells. CAVES are tumor endothelial cells that have broken off from the tumor site into the blood stream. CAVEs are often found in clusters. CAVEs express cytokeratin and various subtypes endothelial cell markers such as CD31, CD146, CD144, CD105, but do not express CD14 or CD45 ^{[20]}.

Recently, another circulating cell associated with cancer has been identified in the peripheral blood of cancer patients. This cancer stromal cell subtype has been termed a cancer associated macrophage-like cell or CAML. CAMLs have been identified in the blood using a non-affinity microfiltration based method which captures both CTCs and CAMLs, and allows for singular or parallel analysis of these cancer specific circulating cell subtypes ^{[1,6-16]}. CAMLs are a recently defined circulating myeloid derived stromal cell, found in all the stages of invasive malignancy and in various solid malignancies (e.g. breast, prostate, non-small cell lung carcinoma (NSCLC), and pancreatic) ^{[11, 13, 14, 17]}. CAMLs are specialized myeloid polyploid cells in the blood in all stages of solid tumors. They are easy to identify by their large size (greater than 25 µm), polyploid nucleus and morphologies: round, rod shaped, with one tail or two tails 180 degrees apart. CAMLs typically express CD31, CD14, CD45 and cytokeratin, and can also express EpCAM, CD146, CD11c and tie2 ^{[11, 13, 14, 17]}.

While the simple presence of one or more of these circulating cell types in a blood sample obtained from a subject having cancer provides diagnostic information, the presence of selected biomarkers in the cells can provide additional information. For example, and as discussed herein, the present inventors have discovered that the level of CCR5 expression in circulating cells, such as CAMLs, CTCs, PDCTCs, apoptotic CTCs, EMTCTCs and CAVEs is associated with overall survival (OS) and progression free survival (PFS) of a subject having cancer. In particular, it was found that high levels of expression of the CCR5 protein receptor correlates with shorter overall survival (OS) and progression free survival (PFS) in a subject having cancer.

### CCR5

CCR5 is a protein receptor expressed on the surface of T cells, macrophages, dendritic cells, eosinophils, microglia and subpopulations of some cancer cells, such as breast cancer and prostate cancer cells. CCR5 plays a vital role in the inflammatory response by directing cells to sites of inflammation. CCR5 has been a key player in HIV-1 entry into target cells. CCR5 has been implicated in other infectious diseases and non-infectious diseases such as cancer, atherosclerosis, and inflammatory bowel disease. CCR5 has been implicated in the development of various types of cancer, including breast cancer, ovarian and cervical cancer, prostate cancer, colorectal cancer, melanoma, pancreatic cancer, Hodgkin lymphoma, multiple myeloma and others ^{[24, 25]}. Approximately 50% of the human breast cancers express CCR5, especially in triple negative breast cancer and most basal breast cancers. Higher cytoplasmic CCR5 staining correlated with poor prognosis ^{[23]}.

CCRS's cognate ligands include CCL3, CCL4, CCL5 and CCL3L1. CCR5 inhibitors and drugs that activate CCR5 antagonists have been shown to reduce metastasis ^{[26]}. In the tumor, activated CCR5 moves into the nucleus. Indeed, activation of CCR5 and subsequent endocytosis of the receptor is indicative of highly aggressive cancer cells that promote metastasis and cause tumor spread. The MDA-MB-231 breast cell line has been used as a model system to visualize activation and internalization of CCR5 via binding of the receptor by its ligand CCL5. CCL5-dependent CCR5 activation and endocytosis is visualized by the formation of endocytic CCR5 endosomes, which have been previously defined as "pools" by Signoret et al. ^{[29]}.

In particular, ligand binding activates cell surface-localized CCR5, leading to its phosphorylation and subsequent endocytosis and translocation from the cell surface to the nucleus from the cell surface ^{[27,29]}. This receptor-mediated endocytosis is the internalization of the activated receptor-ligand complex from the cell surface to within the cell ^{[28]}. The expression level of CCR5 is thus regulated by endocytosis and recycling ^{[29,30]}. At the cell surface, receptors are shown to aggregate together into CCR5 clusters or pools generally ranging in size from nanometer scale to micrometer scale ^{[30-32]}. Such pools can be seen in the Figures as dots that are clear and distinct, or more diffuse in nature. The mechanistic rationale for why these CCR5 pools form and exist is unknown ^{[32]}. β-arrestin binds CCR5 at sites of phosphorylation on the receptor and desensitizes the receptor to further activation ^{[29]}. β-arrestin then enables endocytosis by assisting the binding of the phosphorylated receptor to the clathrin-coated pits ^{[29-30]}. These clathrin-coated pits are then fused to early endosomes which are translocated to the perinuclear space ^{[29]}. Endosomes are vesicle organelles within the cells that carry out the sorting of endocytosed material ^{[28]}. Within the perinuclear endosome, CCR5 is sensitized to its dephosphorylated form and returned to the cell surface ^{[29,30]}. Thus, in each of the embodiments and aspects of the invention, the term or phrase "CCR5 pools" means distinct clusters of CCR5 receptors, whether on the surface of a cell, in the cytoplasm, in the perinuclear space, or in the nucleus.

### Circulating Tumor Cells

As defined herein, CTCs associated with carcinomas express a number of cytokeratins (CKs). CK 8, 18, & 19 are the cytokeratins most commonly expressed and used in diagnostics, but surveying need not be limited to these markers alone. The surface of solid tumor CTCs usually express epithelial cell adhesion molecule (EpCAM). However, this expression is not uniform or consistent. CTCs do not express any CD45 because it is a white blood cell marker. In assays to identify tumor-associated cells, such as CTCs and CAMLs, it is sufficient to use antibodies against markers associated with the solid tumor such as CK 8, 18, & 19, or antibodies against CD45 or DAPI. Combining staining techniques with morphology, pathologically-definable CTCs (PDCTC), apoptotic CTCs and CAMLs can be identified ^{[6]}.

PDCTCs associated with solid tumors express CK 8, 18, & 19, and can be identified and defined by the following characteristics:
- A "cancer-like" nucleus stained by DAPI.
- Expression of one or more of CK 8, 18 and 19; CTCs from epithelial cancers usually express at least CK 8, 18 and 19. The cytokeratins have a filamentous pattern.
- Lack of CD45 expression.

An apoptotic CTCs associated with cancer express CK 8, 18, & 19 and can be identified and defined by the following characteristics:
- A degrading nuclei.
- Expression of one or more of CK 8, 18 and 19; not all the cytokeratins are filamentous in pattern, but parts or whole appear fragmented in the form of spots.
- Lack of CD45 expression.

### Circulating Cancer Associated Macrophage-like Cells (CAMLs)

As defined herein, the circulating cells used in the methods of the invention can be termed "CAMLs". Each reference to "circulating cells" is synonymous with CAMLs, and each reference to "CAMLs" is synonymous with circulating cells. Whether termed CAMLs or "circulating cells" these cells are characterized by having one or more of the following features:
- CAMLs have a large, atypical polyploid nucleus or multiple individual nuclei, often scattered in the cell, though enlarged fused nucleoli are common. CAML nuclei generally range in size from about 10 µm to about 70 µm in diameter, more commonly from about 14 µm to about 64 µm in diameter.
- For many cancers, CAMLs express the cancer marker of the disease. For example, CAMLs associated with epithelial cancers may express CK 8, 18 or 19, vimentin, etc. The markers are typically diffused, or associated with vacuoles and/or ingested material. The staining pattern for any marker is nearly uniformly diffused throughout the whole cell. For sarcomas, neuroblastomas and melanomas, other markers associated with the cancers can be used instead of CK 8, 18, 19.
- CAMLs can be CD45 positive or CD45 negative, and the present invention encompasses the use of both types of CAMLs.
- CAMLs are large, approximately 20 micron to approximately 300 micron in size by the longest dimension.
- CAMLs are found in many distinct morphological shapes, including spindle, tadpole, round, oblong, two legs, more than two legs, thin legs, or amorphous shapes.
- CAMLs from carcinomas typically have diffused cytokeratins.
- If CAMLs express EpCAM, EpCAM is typically diffused throughout the cell, or associated with vacuoles and/or ingested material, and nearly uniform throughout the whole cell, but not all CAML express EpCAM, because some tumors express very low or no EpCAM.
- If CAMLs express a marker, the marker is often diffused throughout the cell, or associated with vacuoles and/or ingested material, and nearly uniform throughout the whole cell, but not all CAML express the same markers with equal intensity and for a limited number of markers, the markers are not distributed equally throughout the cell.
- CAMLs often express markers associated with the markers of the tumor origin; e.g., if the tumor is of prostate cancer origin and expresses PSMA, then CAMLs from such a patient also expresses PSMA. As another example, if the primary tumor is of pancreatic origin and expresses PDX-1, then CAMLs from such a patient also expresses PDX-1. As further example, if the primary tumor or CTC of the cancer origin express CXCR-4, then CAMLs from such a patient also express CXCR-4.
- If the primary tumor or CTC originating from the cancer expresses a biomarker of a drug target, CAMLs from such a patient also express the biomarker of the drug target. An example of such a biomarker of immunotherapy is PD-L1.
- CAMLs express monocytic markers (e.g. CD11c, CD14) and endothelial markers (e.g. CD146, CD202b, CD31).
- CAMLs have the ability to bind Fc fragments.

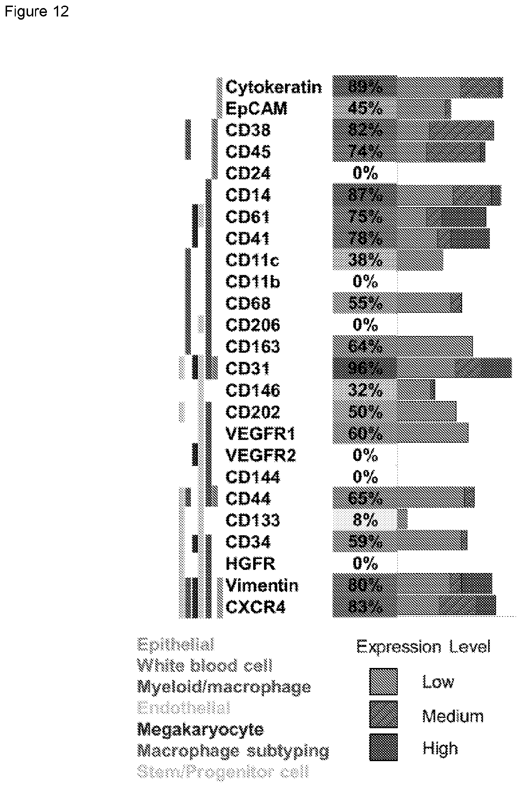

An extensive set of markers were evaluated for their expression on CAMLs, and the results are shown in Figure 12. In one aspect of the invention, the CAMLs of the present invention express 1, 2, 3, 4, 5, 6, 7, 8, 9, 10, 11, 12, 13, 14, 15, 16, 17, 18, 19, 20 or all 21 of the markers shown in Figure 12. The markers were screened against 1118 CAMLs from 93 different patients with different cancers. CAMLs were initially isolated and identified with DAPI, cytokeratin, and CD45; then sequentially restained with total of 27 markers including myeloid/macrophage, white blood cell, megakaryocyte, epithelial, endothelial, progenitor/stem, and motility markers. As can be seen from Figure 12, marker expression ranges from 0% to 96%. Almost all CAMLs were found to express levels of CD31, and commonly co-expressed cytokeratin, CD14, CXCR4, vimentin and other markers. However, while CAMLs contained clear myeloid lineage marker (CD14), CD31 marker was expressed more often at 96%.

CAMLs also present with numerous phenotypes which do not appear to match the understanding of classical cellular differentiation (i.e. co-expression of CD45 [leukocyte] and cytokeratin [epithelial], CD11c/CD14 [macrophage] and CD41 [macrophage/megakaryocyte], CD146 [endothelial] and CD61 [macrophage/endothelial/megakaryocyte], CD31 [white blood cell/macrophage/endothelial/megakaryocyte/stem cell] and CD68/CD163 [macrophage]). Many of the markers appear on multiple cell types. Combined, these data show CAMLs are myeloid-derived cells early in their differentiation process that possess many phenotypic attributes associated with stem cell and proangiogenic capabilities.

CAMLs can be visualized by colorimetric stains, such as H&E, or fluorescent staining of specific markers as shown in Figure 12. For the cytoplasm, CD31 is the most positive phenotype. CD31 alone, or in combination with other positive markers in Figure 12, or cancer markers associated with the tumor are recommended.

Thus, and in the various embodiments and aspects of the invention, the circulating cells (CAMLs) can be defined as having each of the following characteristics:
(a) large atypical polyploid nucleus of about 14-64 µm in size, or multiple nuclei in the same cell;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, the circulating cells (CAMLs) can be further identified as having one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype.

As suggested above, the unique characteristics of the CAMLs and CTCs described herein make them well-suited for use in clinical methodology including methods of screening and diagnosis diseases such as cancer, monitoring treatment, monitoring of disease progression and recurrence.

### Predicting OS and PFS Using Mean and Median Values of CCRS Pools/Clusters

As suggested in the Summary above, the present invention is directed to a method for predicting overall survival (OS) and progression free survival (PFS) of a subject having cancer, said method comprising: determining the number of CCR5 pools/clusters in circulating cells in a biological sample from a first subject having cancer, and comparing the results to those determined from a second subject having the same type of cancer, wherein the OS and PFS of the subject having a greater number of CCR5 pools/clusters is predicted to be shorter than the OS and PFS of the subject having a fewer number of CCR5 pools/clusters; or determining the mean number of CCR5 pools/clusters in a selected number of circulating cells in a biological sample from a first subject having cancer, and comparing the results with those determined from a second subject having the same type of cancer, wherein the OS and PFS of the subject with a greater mean number of CCR5 pools/clusters is predicted to be shorter than the OS and PFS of the subject having a lesser mean number of CCR5 pools/clusters; or determining the median number of CCR5 pools/clusters in a selected number of circulating cells in a biological sample from a first subject having cancer, and comparing the results with those determined from a second subject having the same type of cancer, wherein the OS and PFS of the subject with a greater median number of CCR5 pools/clusters are predicted to be shorter than the OS and PFS of the subject having a lesser median number of CCR5 pools/clusters, wherein the circulating cells comprise cancer associated macrophage-like cells (CAMLs) and wherein the CAMLs have the following characteristics: (a) large atypical polyploid nucleus of about 14-64 µm in size, or multiple nuclei in a single cell; (b) cell size of about 20-300 µm in size; and (c) morphological shape selected from the group consisting of spindle, tadpole, round, oblong, two legs, more than two legs, thin legs, and amorphous; optionally wherein the CAMLs have one or more of the following additional characteristics: (d) CD14 positive phenotype; (e) CD45 expression; (f) EpCAM expression; (g) vimentin expression; (h) PD-L1 expression; (i) CD11C marker expression; (j) CD146 marker expression; (k) CD202b marker expression; (l) CD31 marker expression; and (m) CK8, 18, 19 epithelial phenotype, and wherein the source of the biological sample is one or more of peripheral blood, blood, lymph node, bone marrow, cerebral spinal fluid, tissue, and urine.

When comparing the number of CCR5 pools/clusters in the circulating cells (e.g., CAMLs) between two subjects having cancer, it is preferable for the subjects to have the same type of cancer. However, it may be difficult to fully match two subjects in terms of the type of cancer, the stage of the cancer, the rate of progression of the cancer, history of treatment, and history of remission and/or reoccurrence of the cancer, among other factors. Therefore, it should be understood that there may be some variations in the characteristics in the cancers of two subject that are being compared in this method.

It should also be understood that the data to which the determinations are compared, e.g. the number of CCR5 pools/clusters from a second subject having the same type of cancer, may be data from a single subject, or the combined results of data from two or more subjects. The predictive value of the methods of the invention will be increased over time as baselines are established on data from groups of subjects having the same or similar cancers. Therefore, as used herein, "second subject having the same type of cancer" means a single subject or data from a group of two or more subjects having the same type of cancer.

In these aspects of the invention, the number of circulating cells in which CCR5 pools/clusters are counted can range from 1 to 100 cells, and includes 1 cell, and the ranges of 1-2 cells, 1-3 cells, 1-4 cells, 1-5 cells, 1-6 cells, 1-7 cells, 1-8 cells, 1-9 cells, 1-10 cells, 1-20 cells, 1-30 cells, 1-40 cells, and 1-50 cells.

In each aspect of this embodiment, the circulating cells are CAMLs. In related aspects, the methods can be performed using CAMLs and as well as one or more of CTCs, PDCTCs, apoptotic CTCs, EMTCTCs and CAVEs.

### Predicting OS and PFS Using Number of CCR5 Pools/Clusters

The present invention is also directed to a method for predicting OS and PFS of a subject having cancer, said method comprising determining the number of CCR5 pools/clusters in circulating cells in a biological sample from a subject having cancer, wherein the circulating cells comprise cancer associated macrophage-like cells (CAMLs) and wherein the CAMLs have the following characteristics: (a) large atypical polyploid nucleus of about 14-64 µm in size, or multiple nuclei in a single cell; (b) cell size of about 20-300 µm in size; and (c) morphological shape selected from the group consisting of spindle, tadpole, round, oblong, two legs, more than two legs, thin legs, and amorphous; optionally wherein the CAMLs have one or more of the following additional characteristics: (d) CD14 positive phenotype; (e) CD45 expression; (f) EpCAM expression; (g) vimentin expression; (h) PD-L1 expression; (i) CD11C marker expression; (j) CD146 marker expression; (k) CD202b marker expression; (l) CD31 marker expression; and (m) CK8, 18, 19 epithelial phenotype, wherein the source of the biological sample is one or more of peripheral blood, blood, lymph node, bone marrow, cerebral spinal fluid, tissue, and urine, and wherein when at least one CAML cell in said sample has about 10 or more CCR5 pools/clusters, the OS and PFS of the subject is predicted to be shortened, or the OS and PFS of the subject is predicted to be shorter than a subject having the same type of cancer where none of the cells in a corresponding biological sample has about 10 or more CCR5 pools/clusters.

Generally, when the method is based on identifying at least one cell having 15 or more CCR5 pools/clusters and the biological sample is blood, the volume of the blood sample is about 7.5 ml.

These methods can be performed using the following integers in place of the integer 15 discussed above: 10, 11, 12, 13, 14, 16, 17, 18, 19, 20, 21, 22, 23, 24, 25, 26, 27, 28 29, or 30. For example, the method also comprises determining the number of CCR5 pools/clusters in circulating cells in a biological sample from a subject having cancer, wherein when at least one cell in said sample has about 15 or more CCR5 pools/clusters, the OS and PFS of the subject is predicted to be shortened; or
wherein when at least one cell in said sample has about 16 or more CCR5 pools/clusters, the OS and PFS of the subject is predicted to be shortened; or
wherein when at least one cell in said sample has about 17 or more CCR5 pools/clusters, the OS and PFS of the subject is predicted to be shortened; or
wherein when at least one cell in said sample has about 18 or more CCR5 pools/clusters, the OS and PFS of the subject is predicted to be shortened; or
wherein when at least one cell in said sample has about 19 or more CCR5 pools/clusters, the OS and PFS of the subject is predicted to be shortened; or
wherein when at least one cell in said sample has about 20 or more CCR5 pools/clusters, the OS and PFS of the subject is predicted to be shortened; or
wherein when at least one cell in said sample has about 21 or more CCR5 pools/clusters, the OS and PFS of the subject is predicted to be shortened; or
wherein when at least one cell in said sample has about 22 or more CCR5 pools/clusters, the OS and PFS of the subject is predicted to be shortened; or
wherein when at least one cell in said sample has about 23 or more CCR5 pools/clusters, the OS and PFS of the subject is predicted to be shortened; or
wherein when at least one cell in said sample has about 24 or more CCR5 pools/clusters, the OS and PFS of the subject is predicted to be shortened; or
wherein when at least one cell in said sample has about 25 or more CCR5 pools/clusters, the OS and PFS of the subject is predicted to be shortened.

The method also comprises determining the number of CCR5 pools/clusters in circulating cells in a biological sample from a subject having cancer, wherein when at least one cell in said sample has about 15 or more CCR5 pools/clusters, the OS and PFS of the subject is predicted to be shorter than a subject having the same type of cancer where none of the cells in a corresponding biological sample has about 15 or more CCR5 pools/clusters; or
wherein when at least one cell in said sample has about 16 or more CCR5 pools/clusters, the OS and PFS of the subject is predicted to be shorter than a subject having the same type of cancer where none of the cells in a corresponding biological sample has about 16 or more CCR5 pools/clusters; or
wherein when at least one cell in said sample has about 17 or more CCR5 pools/clusters, the OS and PFS of the subject is predicted to be shorter than a subject having the same type of cancer where none of the cells in a corresponding biological sample has about 17 or more CCR5 pools/clusters; or
wherein when at least one cell in said sample has about 18 or more CCR5 pools/clusters, the OS and PFS of the subject is predicted to be shorter than a subject having the same type of cancer where none of the cells in a corresponding biological sample has about 18 or more CCR5 pools/clusters; or
wherein when at least one cell in said sample has about 19 or more CCR5 pools/clusters, the OS and PFS of the subject is predicted to be shorter than a subject having the same type of cancer where none of the cells in a corresponding biological sample has about 19 or more CCR5 pools/clusters; or
wherein when at least one cell in said sample has about 20 or more CCR5 pools/clusters, the OS and PFS of the subject is predicted to be shorter than a subject having the same type of cancer where none of the cells in a corresponding biological sample has about 20 or more CCR5 pools/clusters; or
wherein when at least one cell in said sample has about 21 or more CCR5 pools/clusters, the OS and PFS of the subject is predicted to be shorter than a subject having the same type of cancer where none of the cells in a corresponding biological sample has about 21 or more CCR5 pools/clusters; or
wherein when at least one cell in said sample has about 22 or more CCR5 pools/clusters, the OS and PFS of the subject is predicted to be shorter than a subject having the same type of cancer where none of the cells in a corresponding biological sample has about 22 or more CCR5 pools/clusters; or
wherein when at least one cell in said sample has about 23 or more CCR5 pools/clusters, the OS and PFS of the subject is predicted to be shorter than a subject having the same type of cancer where none of the cells in a corresponding biological sample has about 23 or more CCR5 pools/clusters; or
wherein when at least one cell in said sample has about 24 or more CCR5 pools/clusters, the OS and PFS of the subject is predicted to be shorter than a subject having the same type of cancer where none of the cells in a corresponding biological sample has about 24 or more CCR5 pools/clusters; or
wherein when at least one cell in said sample has about 25 or more CCR5 pools/clusters, the OS and PFS of the subject is predicted to be shorter than a subject having the same type of cancer where none of the cells in a corresponding biological sample has about 25 or more CCR5 pools/clusters.

In another specific example, the method also comprises determining the number of CCR5 pools/clusters in circulating cells in a biological sample from a subject having cancer, wherein when at least one cell in said sample has about 21 or more CCR5 pools/clusters, the OS and PFS of the subject is predicted to be shortened, and wherein the biological sample is 7.5 ml of blood.

In a specific example, the method also comprises determining the number of CCR5 pools/clusters in circulating cells in a biological sample from a subject having cancer, wherein when at least one cell in said sample has about 21 or more CCR5 pools/clusters, the OS and PFS of the subject is predicted to be shorter than a subject having the same type of cancer where none of the cells in a corresponding biological sample has about 21 or more CCR5 pools/clusters, and wherein the biological sample is 7.5 ml of blood.

To avoid any confusion, the phrase "none of the cells in a corresponding biological sample has about 21 or more CCR5 pools/clusters" should be understood to mean that each of the cells in the biological sample has 20 or fewer CCR5 pools/clusters.

The "21 or more" CCR5 pools/clusters value can be considered a cut-off value for this method. In related aspects of this method, the cut-off value may be any one of 15 or more, 16 or more, 17 or more, 18 or more, 19 or more, 20 or more, 21 or more, 22 or more, 23 or more, 24 or more, or 25 CCR5 pools/clusters or more.

In each of the embodiments and aspects of the invention, the CCR5 pools/clusters may be localized to the nucleus of the cell, or localized to the cytoplasm of the cell, or localized to the surface of the cell, or some combination thereof.

In each of the embodiments and aspects of the invention, the CCR5 of the circulating cells may be in an activated state, or an inactivated state, or the cells may have both activated and inactivated receptors.

In each aspect of this embodiment, the circulating cells are CAMLs. In related aspects, the methods can be performed using CAMLs and one or more of CTCs, PDCTCs, apoptotic CTCs, EMTCTCs and CAVEs.

As used herein, overall survival (OS) means the length of time survived by a subject having cancer from a selected date, such as the date of diagnosis, the date on which treatment began, and the date on which blood is drawn to assess cancer progression.

As used herein, progression free survival (PFS) means the length of time survived by a subject having cancer from a selected date, such as the date on which treatment began or the date on which blood is drawn to assess cancer progression, and where the cancer has not worsened or progressed.

In each of the methods of the invention, OS or PFS, or both, is over a period of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 months, or more. In one aspect of the invention, OS or PFS, or both, is over a period of at least about 24 months.

In each of the methods of the invention, when OS and/or PFS is predicted to be "shortened", OS and/or PFS is shorter in duration that would be the case for a subject having cancer in which the recited number of CCR5 pools/clusters had not been found.

In each of the methods of the invention, it will be apparent that the amount of the biological sample in which the circulating cells (e.g., CAMLs) are assayed can vary. However, the biological sample should generally be at least about 2.5 mL. The amount of biological sample may also be at least about 3, 4, 5, 6, 7, 7.5, 8, 9, 10, 11, 12, 12.5, 13, 14, 15, 16, 17, 17.5, 18, 19, 20, 21, 22, 22.5, 23, 24, 25, 26, 27, 27.5, 28, 29, or 30 mL, or more. The amount of biological sample may also be between about 2.5 and 20 mL, between about 5 and 15 mL, or between about 5 and 10 mL. In one aspect of the invention, the biological sample is about 7.5 mL.

In each of the embodiments and aspects of the invention, the source of the biological sample is one or more of peripheral blood, blood, lymph node, bone marrow, cerebral spinal fluid, tissue, and urine. When the biological sample is blood, the blood may be antecubital-vein blood, inferior-vena-cava blood femoral vein blood, portal vein blood, or jugular-vein blood, for example. The sample may be a fresh sample or a cryo-preserved sample that is thawed ^{[14]}.

When comparing the number of CCR5 pools/clusters in the circulating cells (e.g., CAMLs) between two subjects having cancer, it is preferable for the subjects to have the same type of cancer. However, it may be difficult to fully match two subjects in terms of the type of cancer, the stage of the cancer, the rate of progression of the cancer, history of treatment, and history of remission and/or reoccurrence of the cancer, among other factors. Therefore, it should be understood that there may be some variations in the characteristics in the cancers of two subject that are being compared in this method.

It should also be understood that the data to which the determinations are compared, e.g. the number of CCR5 pools/clusters from a subject having the same type of cancer, may be data from a single subject, or the combined results of data from two or more subjects. The predictive value of the methods of the invention will be increased over time as baselines are established on data from groups of subjects having the same or similar cancers. Therefore, as used herein, "a subject having the same type of cancer" means a single subject or data from a group of two or more subjects having the same type of cancer.

In each of the embodiments and aspects of the invention, the cancer is cancer where the cells thereof express CCR5. The CCR5-expressing cancers may be a solid tumor, Stage I cancer, Stage II cancer, Stage III cancer, Stage IV cancer, carcinoma, sarcoma, neuroblastoma, melanoma, epithelial cell cancer, lung cancer, breast cancer, prostate cancer, pancreatic cancer, melanoma, bladder cancer, kidney cancer, head and neck cancer, colorectal cancer, liver cancer, ovarian cancer, neuroblastoma, sarcoma, osteosarcoma, esophageal, brain & ONS, larynx, bronchus, oral cavity and pharynx, stomach, testis, thyroid, uterine cervix, uterine corpus cancer or other solid tumor cancer. The skilled artisan will appreciate that the methods of the invention are not limited to particular forms or types of cancer and that they may be practiced in association with a wide variety of cancers

In each of the embodiments and aspects of the invention, the circulating cells (e.g., CAMLs) are isolated from the biological samples for the determining steps using one or more means selected from size exclusion methodology, immunocapture, red blood cell lysis, white blood cell depletion, FICOLL, electrophoresis, dielectrophoresis, flow cytometry, magnetic levitation, and various microfluidic chips, slits, channels, hydrodynamic size-based sorting, grouping, trapping, concentrating large cells, eliminating small cells, or a combination thereof. In a particular aspect, the size exclusion methodology comprises use of a microfilter.

In one aspect of the invention, circulating cells are isolated from the biological samples using size exclusion methodology that comprises using a microfilter. Suitable microfilters can have a variety of pore sizes and shapes. The microfilter may have a pore size ranging from about 5 microns to about 20 microns. In certain aspects of the invention, the pore size is between about 5 and 10 microns; in other aspects, the pore size is between about 7 and 8 microns. The larger pore sizes will eliminate most of the WBC contamination on the filter. The pores of the microfilter may have any shape, with acceptable shapes including round, race-track shape, oval, slit, square, rectangular and/or other shapes. The microfilter may have precision pore geometry, uniform pore distribution, more than one pore geometry, and/or non-uniform distribution. The microfilter may be single layer, or multi-layers with different shapes on different layers.

In another aspect of the invention, circulating cells are isolated from the biological samples using a microfluidic chip via physical size-based sorting, slits, channels, hydrodynamic size-based sorting, grouping, trapping, immunocapture, concentrating large cells, or eliminating small cells based on size. The circulating cell capture efficiency can vary depending on the collection method. The size of a circulating cell that can be captured on different platforms can also vary depending, for example, on the identity of the cell. Collection of circulating cells using CellSieve^{™} microfilters provides 100% capture efficiency and high quality cells.

In another aspect of the invention, when the biological sample is peripheral blood or blood, the sample may be collected from a subject using a blood collection tube. CellSave blood collection tubes (Menarini Silicon Biosystems Inc., San Diego, CA), for example, provide stable cell morphology and size.

In another aspect of the invention, circulating cells can be captured and analyzed without specifically identifying the cells as CAML cells *per se.* Instead, one may simply identify the cells based on size of the cytoplasm and nucleus. Examples for doing so are techniques using color metric stains, such as H&E stains, or just looking at CK (+) cells.

In a further aspect of the invention, circulating cells are isolated from the biological samples using a low-pressure microfiltration assay.

In each of the embodiments and aspects of the invention, the number of CCR5 pools/clusters in a cell can be determined by means that include contacting the cells with a labeled marker that has binding specificity for CCR5, and then detecting the marker. Such markers include anti-CCR5 antibodies conjugated to a detectable label. Suitable antibodies include, but are not limited to, R&D Systems MAB183 clones 45549, and 45531, 45523. Suitable labels that may be conjugated to the antibodies include, but are not limited to, FITC, PE, Cyanine, Alexa Fluor, and DyLight. Suitable means for detecting the labeled markers include, but are not limited to, a fluorescent microscope and a standard light microscope for IHC colorimetric staining. The CCR5 pools/clusters are generally imaged in a fluorescent microscope at 40x magnification.

The subjects mentioned in the methods of the present invention will be a human, a non-human primate, bird, horse, cow, goat, sheep, a companion animal, such as a dog, cat or rodent, or other mammal. The subject having cancer may be undergoing treatment for the cancer. Such treatments include, but are not limited to targeted agents, chemotherapy, and radiation therapy.

### Companion Diagnostics

The information obtained using the methods of the invention, i.e. the expression, intensity and/or number of CCR5 pools/clusters in selected populations of circulating cells in a subject, can be used as a companion or complementary diagnostic. A companion or complementary diagnostic is a diagnostic test that can be used in combination with a therapeutic drug for a selected disease or condition. The companion diagnostic determines the suitability of the drug for treatment of the disease or condition in a particular patient, i.e. the companion diagnostic can help to predict whether the subject will be a responder or non-responder to the effects of the drug on the disease or condition in the subject. Thus, the companion diagnostic provides information that is used in treatment decisions and that is essential for the safe and effective use of a corresponding drug or biological product.

The levels of CCR5 expression in circulating cells can be used as an indicator of a patient's potential to respond to drugs targeting CCR5, such as Leronlimab. For example, patients with low or no CCR5 expression on circulating cells are not expected to benefit from drugs such as Leronlimab. Thus, the expression level of CCR5 on circulating cells can be used as a companion or complementary diagnostic for drugs targeting CCR5 in cancer therapy, and determining levels of CCR5 in circulating cells per the methods of the present invention can be used in making treatment decisions for the subject to which the methods of the present invention are applied. The determination of level of expression also involve the determination of the intensity of the fluorescence of the dye conjugated to the CCR5 antibody on the CCR5 pools/clusters.

Companion or complementary diagnostics often require tissue. Chemotherapeutic therapy and radiation therapy can potentially alter the levels of CCR5 expression on tumor cells. The circulating cells in blood provide the ability to analyze CCR5 expression and to monitor expression over time.

### Examples

### Sample collection and processing

Blood samples (7.5 mL) collected in CellSave preservative tubes^{™} were processed with a CellSieve^{™} Microfiltration Assay using a low-pressure vacuum system ^{[1, 12]} or syringe pump ^{[12]}. The CellSieve^{™} Microfiltration Assay isolates circulating cells based on size exclusion, >7 micron. A trained cytologist identified prognostically relevant pathologically definable CTCs (PDCTCs), EMTCTCs and CAMLs based on morphological features and the phenotypic expression of CD45, EpCAM, Cytokeratins 8, 18, 19 and DAPI ^{[1, 6, 12]} using preestablished cytological features described ^{[6, 11, 14]}. An Olympus BX54WI Fluorescent microscope with Carl Zeiss AxioCam and Zen2011 Blue (Carl Zeiss) was used for all imaging.

### Enumerating PDCTC/EMTCTC subtypes and CAMLs

The defining characteristics of the two most common CTC subtypes found in cancer patients (PDCTCS and EMTCTCs) and those for CAML identification were previously described ^{[1, 6, 10-14]}. For this study only intact PDCTCs, EMTCTCs, and CAMLs were characterized ^{[1, 6, 10-14]}. PDCTCs are CD45 negative, with filamentous cytokeratin positivity and DAPI positive nuclei with malignant pathological criteria, classified as the CellSearch^{®} subtype of CTC ^{[1, 6, 10-14]}. EMTCTCs are CD45 negative with a diffuse and often low cytokeratin signal and a DAPI positive nucleus with abnormal criteria, as previously defined ^{[1, 6-9, 12, 13]}. CAMLs are described as enlarged (>25 µm), multinuclear cells with diffuse cytoplasmic cytokeratin staining, and/or one or more of CD45+, CD14+, and CD31+ ^{[6, 11, 14, 17-19]}. All 3 cell types were identified and imaged by a trained cytologist and confirmed by a pathologist. Apoptotic CTCs and CTCs that could not be cytologically classified as previously described were not included. After identification, cells were imaged and x-y axis of each cell was marked for future analysis. Samples were archived at 4°C for 1-3 years.

### QUAS-R quenching and restaining

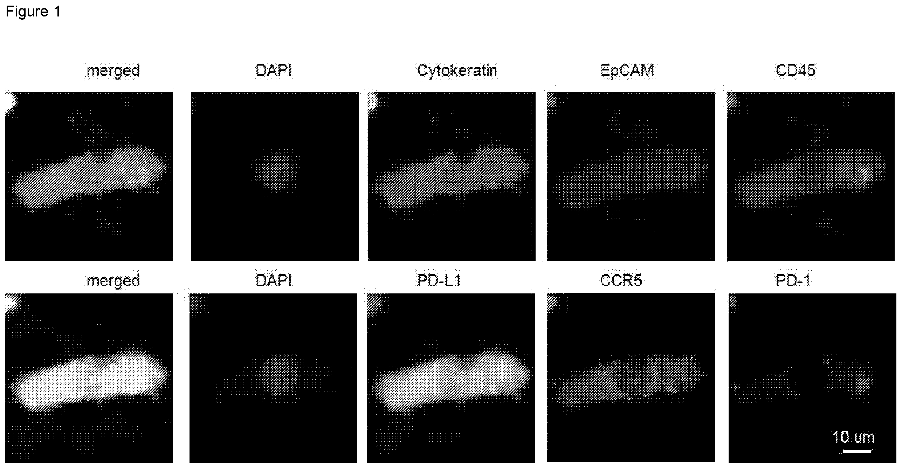

After initial identification and quantification of PDCTCs, EMTCTCs and CAMLs, fluorescence was quenched and samples were restained with CCR5. In some cases, the CCR5 marker is stained alongside a variety of other markers, such as PD-L1 and PD-1. The QUAS-R (Quench, Underivatize, Amine-Strip and Restain) technique was used as previously described ^{[13]}. Briefly, after samples were imaged and marked, filters were subjected to a sequential chemical treatment of quenching solution, Tris, and wash steps. After chemical quenching, filters were washed with PBS, incubated with 1XPBS/20%FBS and then incubated with antibodies against CCR5 for 1 hour at room temp (Figure 1). After antibody incubation, filters are washed in 1xPBST and slide mounted with Fluoromount-G/DAPI (Southern Biotech). Samples were oriented along the x/y axis and previously imaged cells were relocated using a Zen2011 Blue (Carl Zeiss) mark and find software. A Zen2011 Blue (Carl Zeiss) was used to process the images.

### Quantifying CCR5 in Circulating Cells

CCR5 expression using R&D Systems MAB183 clone # 45549 antibodies were analyzed according to manufacturer's guidelines.

### Results

Most of the images of cancer associated circulating cells in blood shown in the Figures are from breast cancer patients in Stages III and IV. Each sample had CAMLs, but only roughly half of the patients had CTCs.

Figure 1 shows a CAML cell initially stained with DAPI, Cytokeratin 8, 18 & 19, EpCAM and CD45 (top row). The second row of Figure 1 shows the cell was restained for DAPI, PD-L1, CCR5 and PD-1. In this example, both PD-L1 and CCR5 expressions are high indicating that a combination of immunotherapies that target these two proteins might be a suitable treatment for this patient. CCR5 is in the cytoplasm; it was not translocated into the nucleus, indicating it was in an active form.

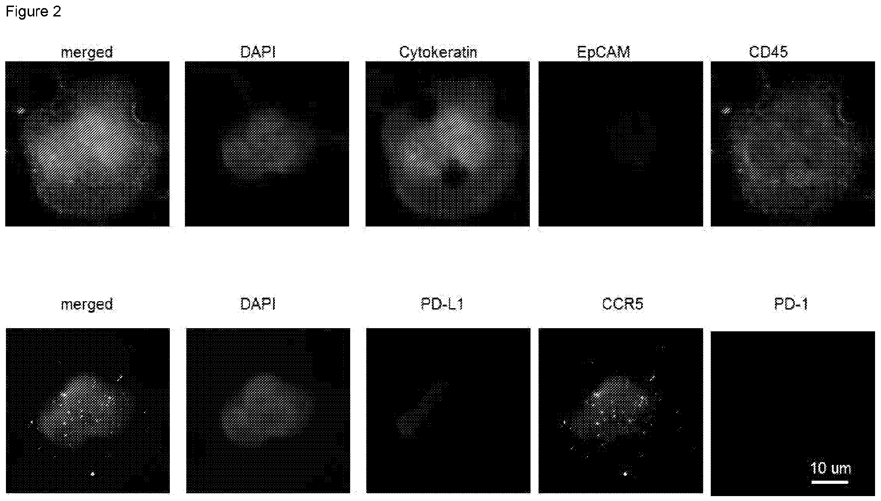

Figure 2 shows a CAML cell initially stained with DAPI, Cytokeratin 8, 18 & 19, EpCAM and CD45 (top row). The second row of Figure 2 shows the cell was restained for DAPI, PD-L1, CCR5 and PD-1. In this example, there is CCR5 expression but no PD-L1 expression, indicating that an immunotherapies alone will not be effective, while therapy targeting CCR5 might be a suitable treatment for this patient. CCR5 pools/clusters are in the cytoplasm; it was not translocated into the nucleus, indicating it was in an active form.

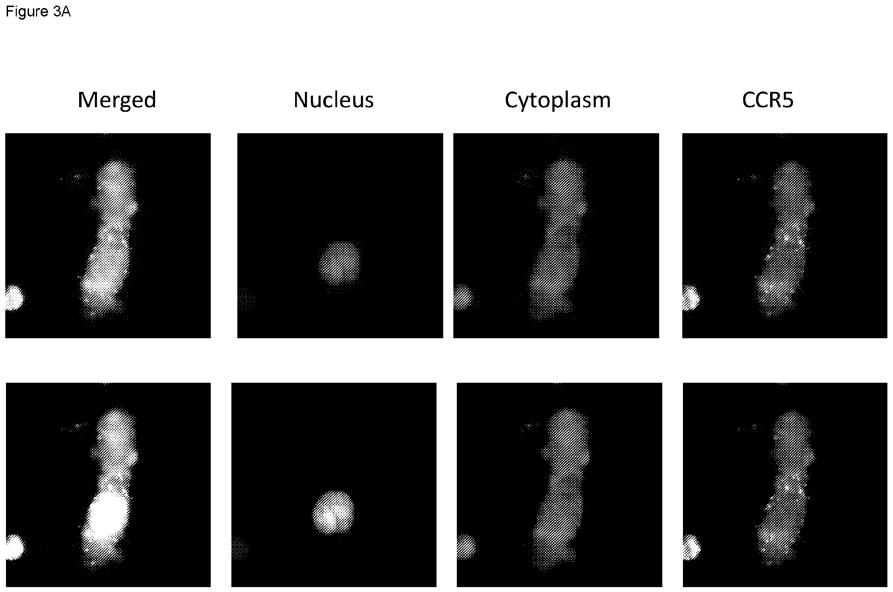

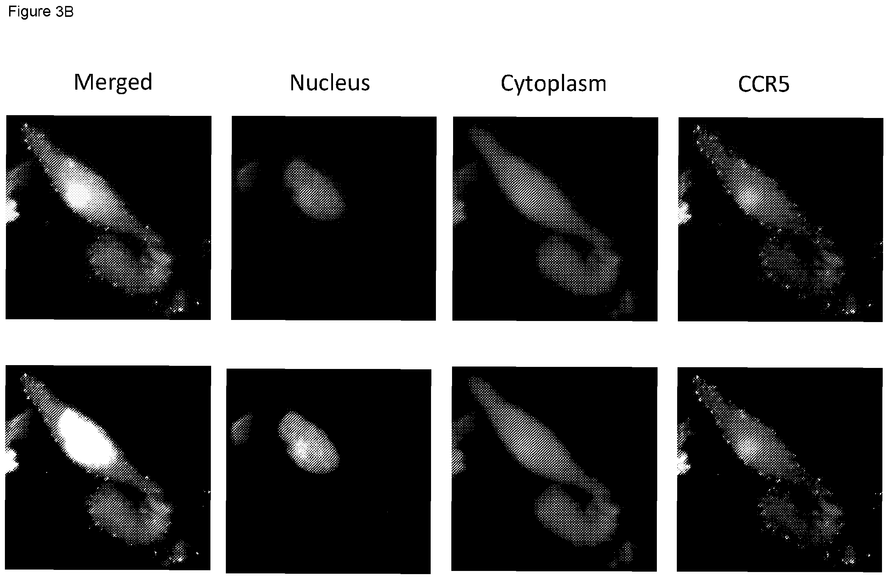

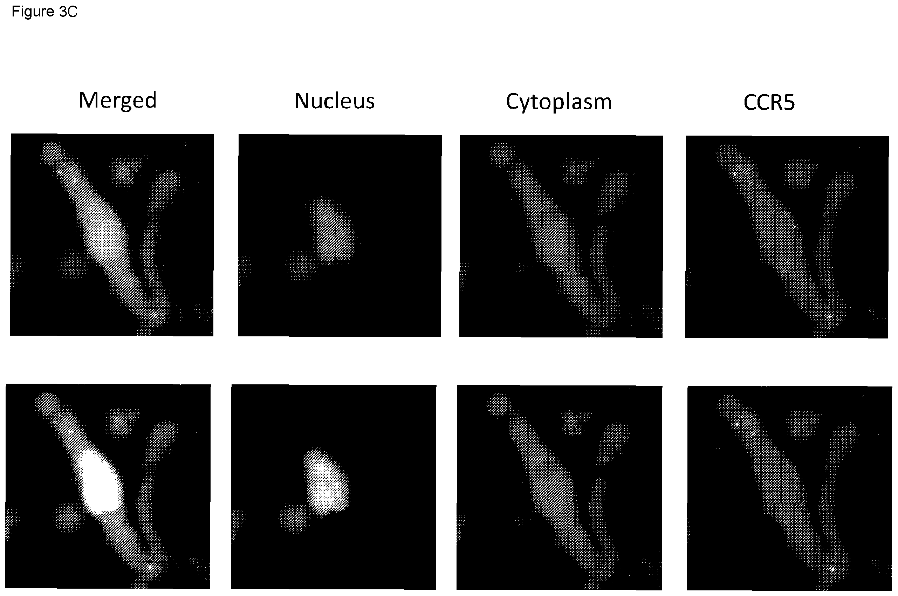

Figures 3-10 focus on CCR5 expression. Black & white and color versions of these figures are provided. Color images allow for the visualization of translocation into the nucleus. For the color images, the nuclei are red, cytoplasm is blue, and CCR5 staining is yellow.

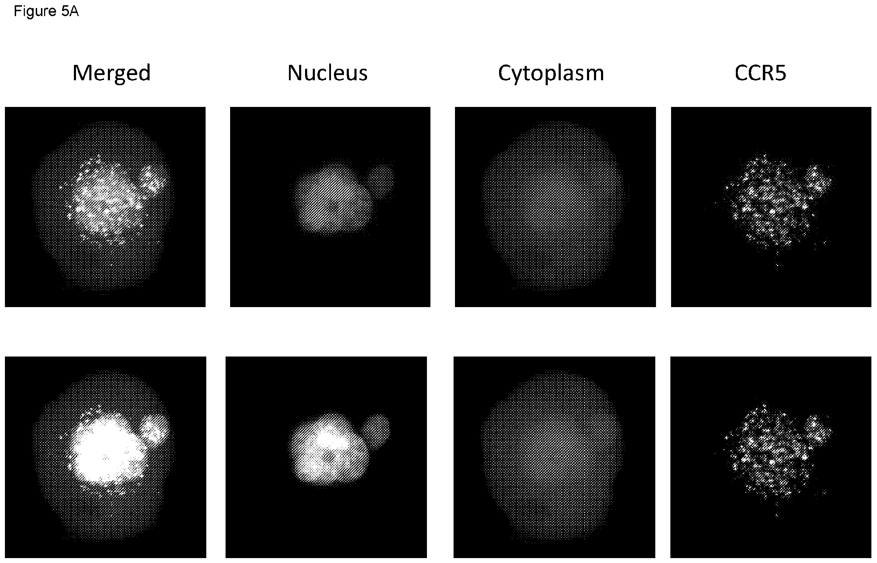

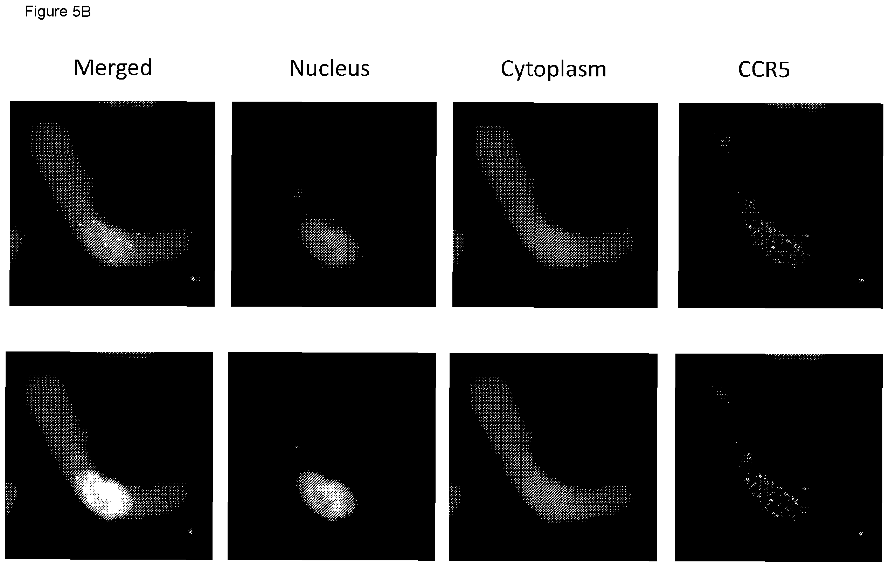

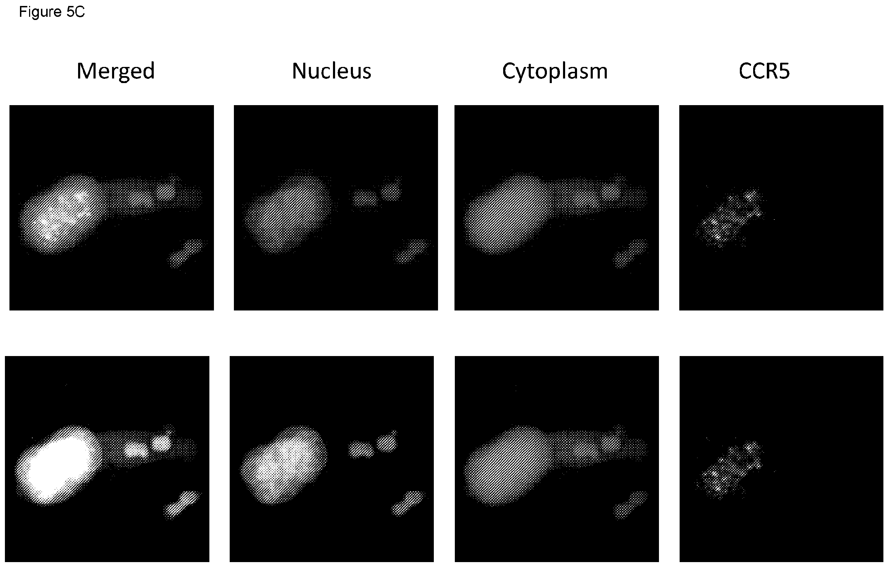

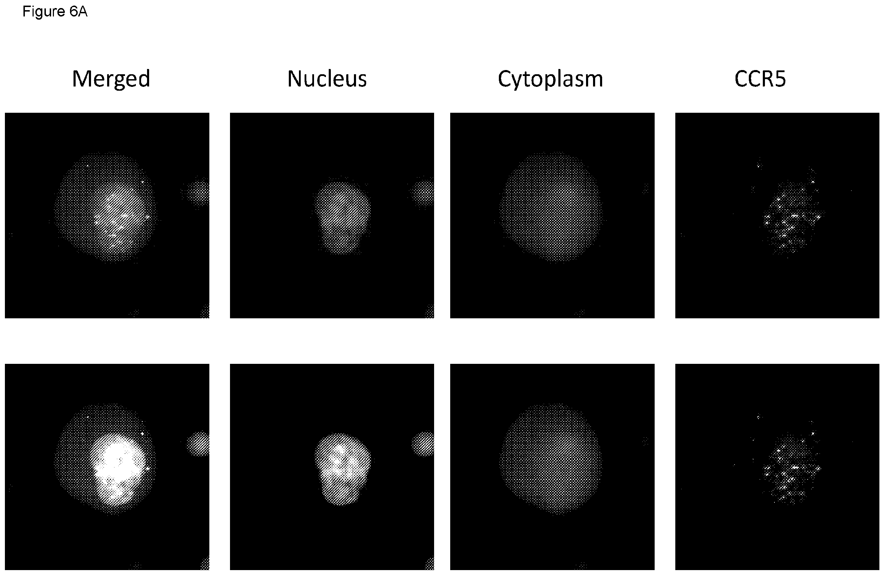

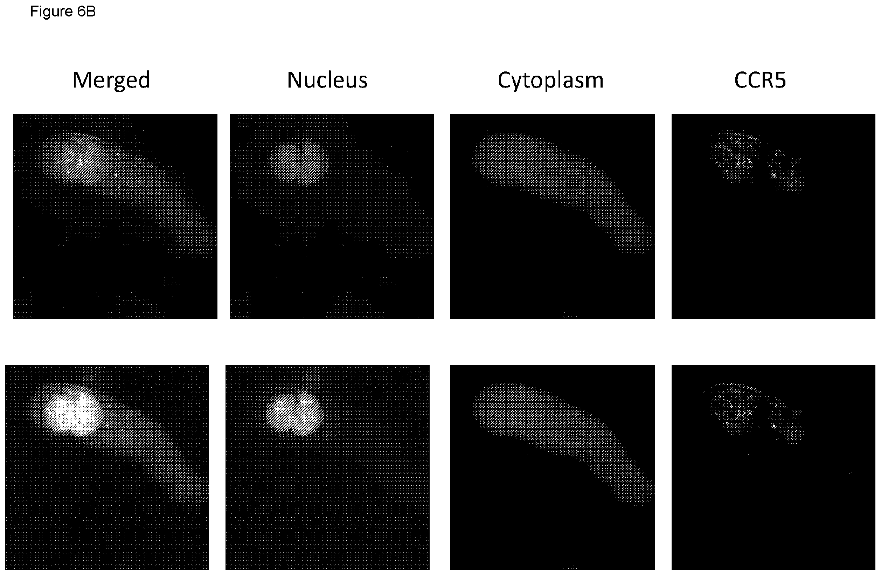

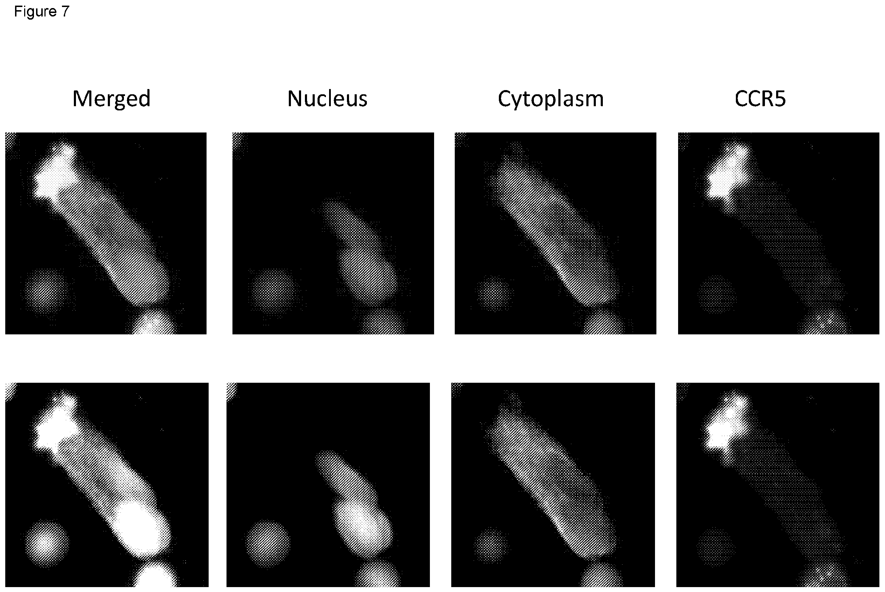

The image sizes for Figures 3, 4, and 6 are 60 µm x 60 µm. The image size for Figure 5A is 60 µm x 60 µm; Figure 5B is 90 µm x 90 µm; Figure 5C is 60 µm x 60 µm; Figure 6A is 60 µm x 60 µm; Figure 6B is 75 µm x 75 µm. The image size for Figures 7-10 are 30 µm x 30 µm.

Figures 3A-C are CAMLs with high/medium CCR5 expression in the form of pools/clusters found on the surface of the cell and in the cytoplasm, indicating the protein is in an activated form.

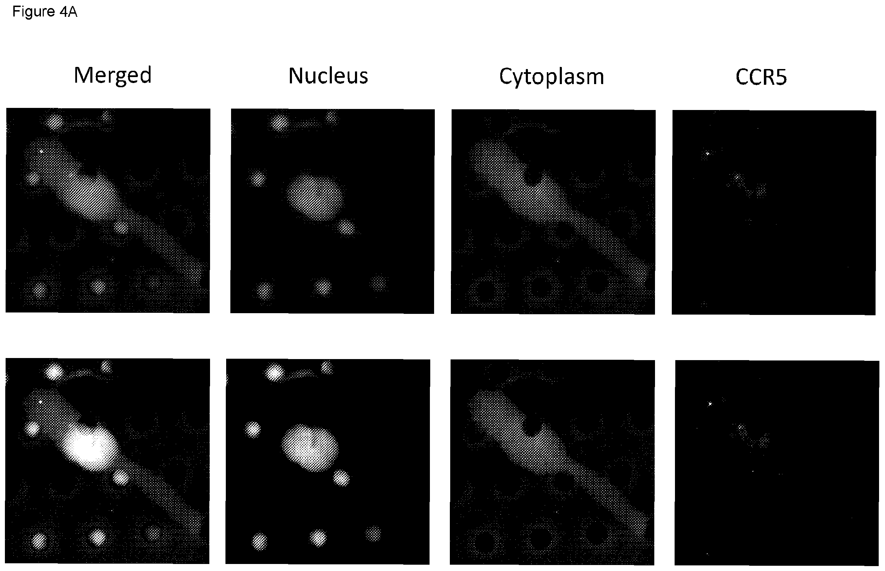

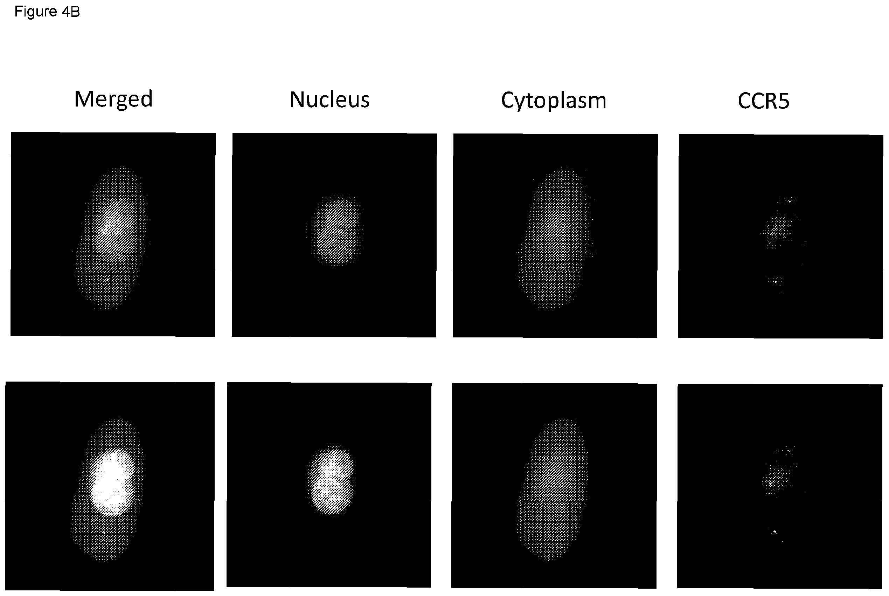

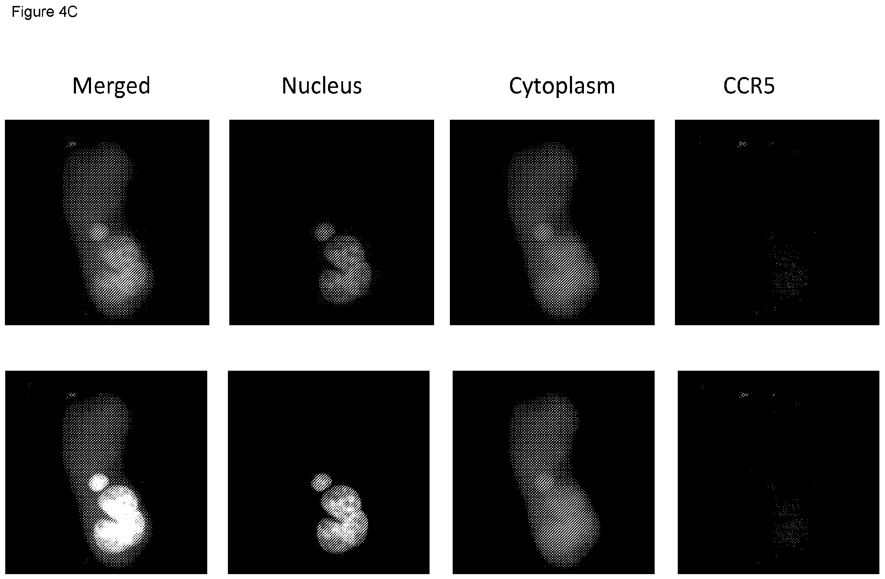

Figures 4A-C are CAMLs with low/negative CCR5 expression in the form of pools/clusters found on the surface of the cell and in the cytoplasm, indicating the protein is in an activated form.

Figures 5A-C are CAMLs with high CCR5 expression in the form of pools/clusters, with most pools/clusters translocated into the nucleus, indicating the protein is in an inactivated form. Figure 5A shows high expression of CCR5 in the cytoplasm. Figure 5B shows CCR5 has mostly translocated into the nucleus. Figure 5C shows CCR5 has completely translocated into the nucleus.

Figures 6A-B are CAMLs with medium/low CCR5 expression in the form of pools/clusters, with most pools/clusters translocated into the nucleus in Figure 6B, indicating the protein is in an inactivated form.

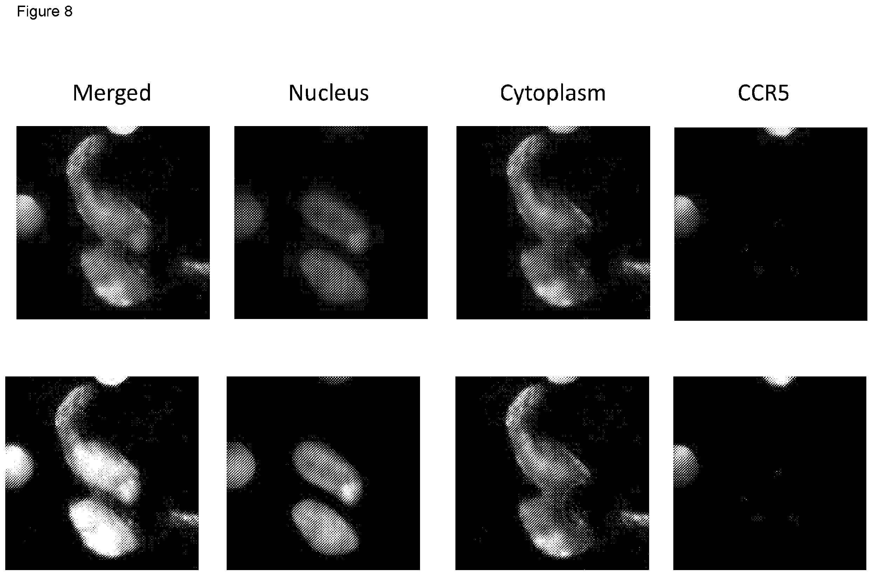

CCR5 pools/clusters are found on other cancer associated cells such as EMTCTCs and CTCs. Figure 7 shows EMTCTCs with localized high CCR5 expression aggregated to one spot on the cell surface. Figure 8 shows EMTCTCs without CCR5 expression.

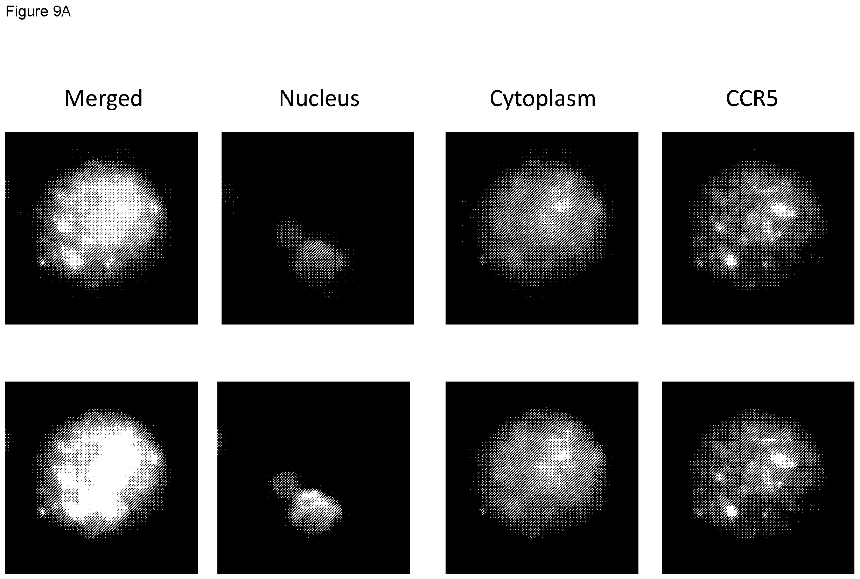

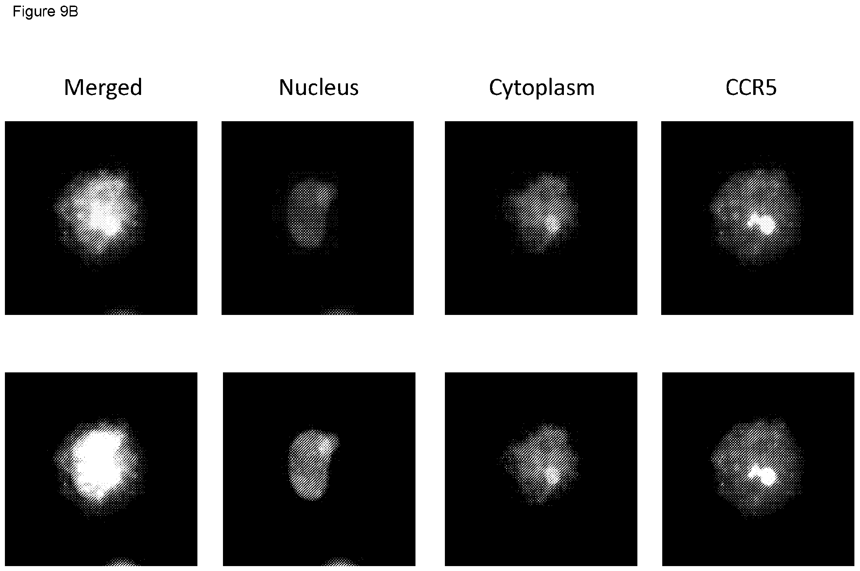

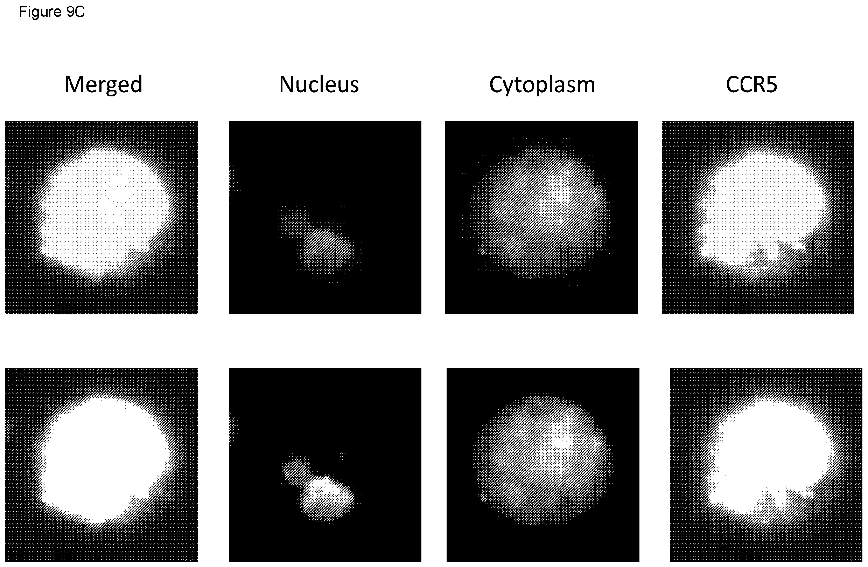

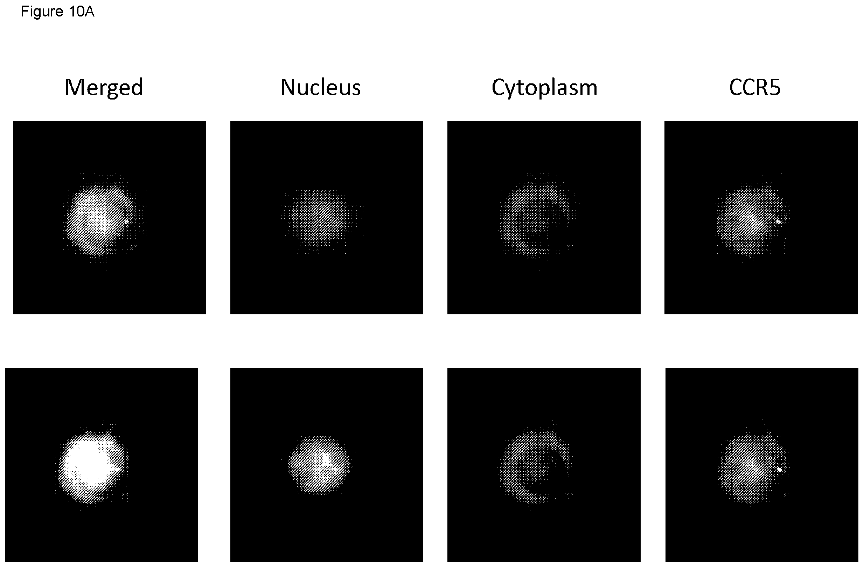

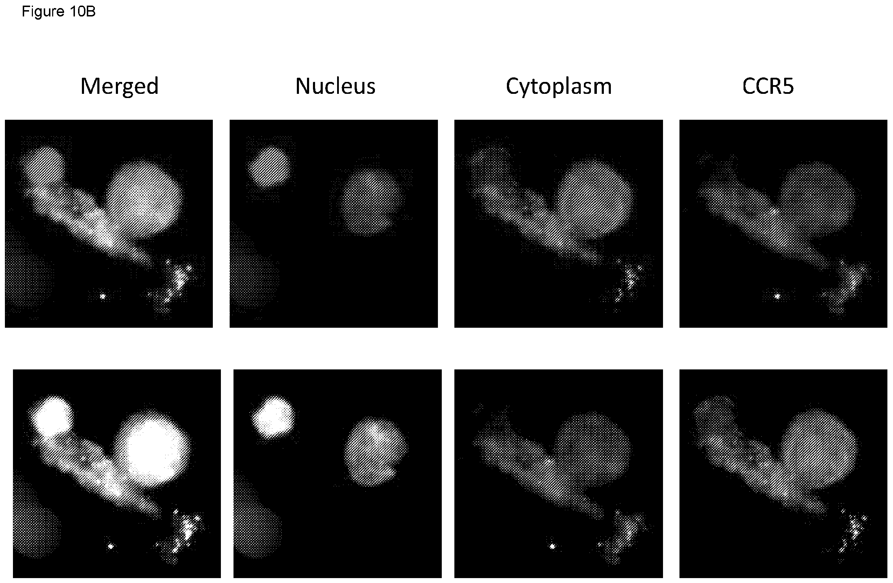

Figures 9A-C show CTCs with moderate or high CCR5 expression. The amount of CCR5 in Figure 9C is significantly higher than usual. Figures 10A-B are CTCs with low expressions of CCR5 with only a few small pools/clusters.

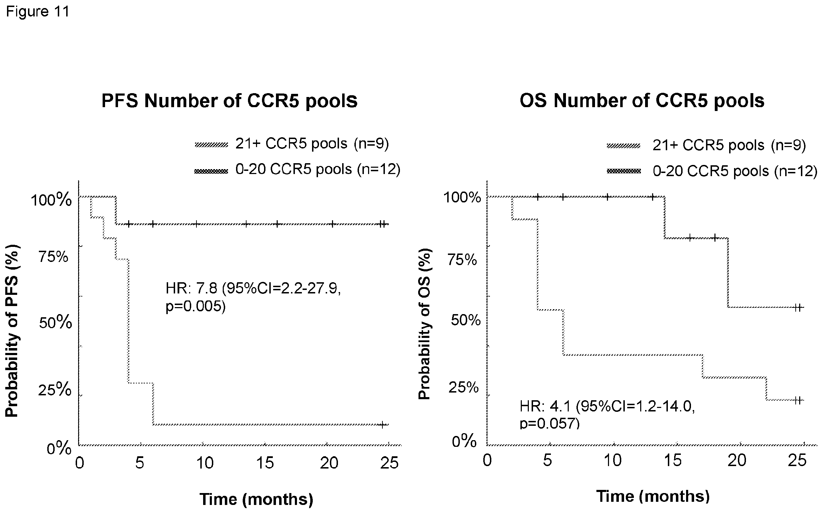

Figure 11 shows the importance of the pools/clusters or pools/clusters based on 21 breast cancer patients in Stages III and IV. If the patient has a CAML where the number of CCR5 pools/clusters is greater than or equal to 21, the patient had very poor prognosis. For progression free survival (PFS), the hazard ratio (HR) is 7.8 (95%CI=2.2-27.9, p=0.005). For overall survival (OS), the hazard ratio is 4.1 (95%CI=1.2-14.0, p=0.057). CCR5 can be activated or inactivated, i.e. localized to either the nucleus or the cytoplasm.

The cutoff of 20 CCR5 pools/clusters or pools/clusters used in Figure 11 is an example providing the best p-value for the 21 breast cancer patients.

### Statistical Methods

Kaplan-Meier analyses were done in MATLAB R2013A using the counts from all subtypes and the known patient populations. For progression free survival analysis, the time to progression was defined as the interval between when T0 blood sample was obtained to date of progression, all patients remained on study through 24 month end point, i.e. no patients were censored.

### CITATIONS

1. Adams DL, Zhu P, Makarova OV, Martin SS, Charpentier M, Chumsri S, et al. The systematic study of circulating tumor cell isolation using lithographic microfilters. RSC Advances. 2014;4:4334-42.
2. Lianidou ES, Markou A. Circulating tumor cells in breast cancer: detection systems, molecular characterization, and future challenges. Clinical chemistry. 2011;57:1242-55.
3. Pantel K, Brakenhoff RH, Brandt B. Detection, clinical relevance and specific biological properties of disseminating tumour cells. Nature reviews Cancer. 2008;8:329-40.
4. Paterlini-Brechot P, Benali NL. Circulating tumor cells (CTC) detection: clinical impact and future directions. Cancer letters. 2007;253:180-204.
5. Adams D, Tsai S, Makarova OV, Zhu P, Li S, Amstutz PT, et al. Low cytokeratin-and low EpCAM-expressing circulating tumor cells in pancreatic cancer. ASCO Annual Meeting Proceedings; 2013. p. 11046.
6. Adams DL, Stefansson S, Haudenschild C, Martin SS, Charpentier M, Chumsri S, et al. Cytometric characterization of circulating tumor cells captured by microfiltration and their correlation to the cellsearch((R)) CTC test. Cytometry Part A : the journal of the International Society for Analytical Cytology. 2015;87:137-144.
7. Krebs MG, Hou JM, Sloane R, Lancashire L, Priest L, Nonaka D, et al. Analysis of circulating tumor cells in patients with non-small cell lung cancer using epithelial marker-dependent and -independent approaches. J Thorac Oncol. 2012;7:306-15.
8. Farace F, Massard C, Vimond N, Drusch F, Jacques N, Billiot F, et al. A direct comparison of CellSearch and ISET for circulating tumour-cell detection in patients with metastatic carcinomas. British journal of cancer. 2011;105:847-53.
9. Lecharpentier A, Vielh P, Perez-Moreno P, Planchard D, Soria JC, Farace F. Detection of circulating tumour cells with a hybrid (epithelial/mesenchymal) phenotype in patients with metastatic non-small cell lung cancer. British journal of cancer. 2011;105:1338-41.
10. Adams DL, Adams DK, Stefansson S, Haudenschild C, Martin SS, Charpentier M, et al. Mitosis in circulating tumor cells stratifies highly aggressive breast carcinomas. Breast cancer research : BCR. 2016;18:44.
11. Adams DL, Adams DK, Alpaugh RK, Cristofanilli M, Martin SS, Chumsri S, et al. Circulating Cancer-Associated Macrophage-Like Cells Differentiate Malignant Breast Cancer and Benign Breast Conditions. Cancer Epidemiol Biomarkers Prev. 2016;25:1037-42.
12. Adams DL, Alpaugh RK, Martin SS, Charpentier M, Chumsri S, Cristofanilli M, et al. Precision microfilters as an all in one system for multiplex analysis of circulating tumor cells. RSC Advances. 2016;6:6405-14.
13. Adams DL, Alpaugh RK, Tsai S, Tang CM, Stefansson S. Multi-Phenotypic subtyping of circulating tumor cells using sequential fluorescent quenching and restaining. Sci Rep. 2016;6:33488.
14. Adams DL, Martin SS, Alpaugh RK, Charpentier M, Tsai S, Bergan RC, et al. Circulating giant macrophages as a potential biomarker of solid tumors. Proceedings of the National Academy of Sciences of the United States of America. 2014;111:3514-9.
15. Allard WJ, Matera J, Miller MC, Repollet M, Connelly MC, Rao C, et al. Tumor cells circulate in the peripheral blood of all major carcinomas but not in healthy subjects or patients with nonmalignant diseases. Clin Cancer Res. 2004;10:6897-904.
16. Anantharaman A, Friedlander T, Lu D, Krupa R, Premasekharan G, Hough J, et al. Programmed death-ligand 1 (PD-L1) characterization of circulating tumor cells (CTCs) in muscle invasive and metastatic bladder cancer patients. BMC Cancer. 2016;16:744.
17. Mu Z, Benali-Furet N, Uzan G, Znaty A, Ye Z, Paolillo C, et al. Detection and Characterization of Circulating Tumor Associated Cells in Metastatic Breast Cancer. Int J Mol Sci. 2016;17.
18. Lin SH, He J, Edelman M, Xu T, Gao H, Reuben J, et al. Sequential Assessment of DNA Damage Response and PD-L1 Expression in Circulating Tumor Cells of Lung Cancer Patients during Radiotherapy. JOURNAL OF THORACIC ONCOLOGY; 2015: ELSEVIER SCIENCE INC 360 PARK AVE SOUTH, NEW YORK, NY 10010-1710 USA. p. S266-S7.
19. Zhu P, Stanton ML, Castle EP, Joseph RW, Adams DL, Li S, et al. Detection of tumor-associated cells in cryopreserved peripheral blood mononuclear cell samples for retrospective analysis. J Transl Med. 2016;14:198.
20. Daniel L. Adams, R. Katherine Alpaugh, Steven H. Lin, Jeffrey R. Marks, Raymond Bergan, Stuart S. Martin, Saranya Chumsri, Massimo Cristofanilli, Cha-Mei Tang, Steingrimur Stefansson, "Multiplex phenotyping of circulating cancer associated macrophage-like cells in patients with solid tumors", Proceedings of AACR, Vol. 58, April 2017. Abstract #778.
21. International Patent Application Publication No. WO 2013/181532, dated December 5, 2013.
22. International Patent Application Publication No. WO 2016/33103, dated March 3, 2016.
23. Jiao et al., CCR5 Governs DNA Damage Repair and Breast Cancer Stem Cell Expansion. Cancer Res. 78(7):1657-1671 (2018).
24. Vangelista, L. and S. Vento. The Expanding Therapeutic Perspective of CCR5 Blockade. Frontiers in Immunology 8: Article 1981 (2018).
25. Singh et al. CCR5/CCL5 axis interaction promotes migratory and invasiveness of pancreatic cancer cells. Nature Scientific Reports 8:1323, 12 pages (2018).
26. Velasco-Velazquez et al. The potential to target CCL5/CCR5 in breast cancer. Expert Opin. Ther. Targets 18(11):1265-1275 (2014).
27. Cooper GM. The Cell: A Molecular Approach. 2nd edition. Sunderland (MA): Sinauer Associates (2000). Endocytosis.
28. Oppermann M. Chemokine receptor CCR5: insights into structure, function, and regulation. Cell Signal. 16:1201-1210 (2004).
29. Signoret, N., Christophe, T., Oppermann, M., and Marsh, M. pH-independent endocytic cycling of the chemokine receptor CCR5. Traffic 5, 529-543 (2004).
30. Berro, R. et al. Multiple CCR5 conformations on the cell surface are used differentially by human immunodeficiency viruses resistant or sensitive to CCR5 inhibitors. J. Virol. 85, 8227-8240 (2011).
31. Fox JM, Kasprowicz R, Hartley O, Signoret N. CCR5 susceptibility to ligand-mediated down-modulation differs between human T lymphocytes and myeloid cells. J Leukoc Biol. 98:59-71 (2015).
32. Khalid A, Wolfram J, Ferrari I, Mu C, Mai J, Yang Z, Zhao Y, Ferrari M, Ma X, Shen H. Recent advances in discovering the role of CCL5 in metastatic breast cancer. Mini Rev Med Chem. 15:1063-1072 (2015).

## Claims

1. A method for predicting overall survival (OS) and progression free survival (PFS) of a subject having cancer, said method comprising determining the number of CCR5 clusters in circulating cells in a biological sample from a subject having cancer,
wherein the circulating cells comprise cancer associated macrophage-like cells (CAMLs) and wherein the CAMLs have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous; optionally wherein the CAMLs have one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype,
wherein the source of the biological sample is one or more of peripheral blood, blood, lymph node, bone marrow, cerebral spinal fluid, tissue, and urine, and
wherein when at least one CAML cell in said sample has about 10 or more CCR5 clusters:
(i) the OS and PFS of the subject is predicted to be shortened, or
(ii) the OS and PFS of the subject is predicted to be shorter than a subject having the same type of cancer where none of the cells in a corresponding biological sample has about 10 or more CCR5 clusters.

2. A method for predicting OS and PFS of a subject having cancer, said method comprising:
(i) determining the number of CCR5 clusters in circulating cells in a biological sample from a first subject having cancer, and comparing the results to those determined from a second subject having the same type of cancer, wherein the OS and PFS of the subject having a greater number of CCR5 clusters is predicted to be shorter than the OS and PFS of the subject having a fewer CCR5 clusters;
(ii) determining a mean number of CCR5 clusters in a selected number of circulating cells in a biological sample from a first subject having cancer, and comparing the results to those determined from a second subject having the same type of cancer, wherein the OS and PFS of the subject with a greater mean number of CCR5 clusters is predicted to be shorter than the OS and PFS of the subject having a lesser mean number of CCR5 clusters; or
(iii) determining a median number of CCR5 clusters in a selected number of circulating cells in a biological sample from a first subject having cancer, and comparing the results to those determined from a second subject having the same type of cancer, wherein the OS and PFS of the subject with a greater median number of CCR5 clusters is predicted to be shorter than the OS and PFS of the subject having a lesser median number of CCR5 clusters,
wherein the circulating cells comprise cancer associated macrophage-like cells (CAMLs) and wherein the CAMLs have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous; optionally wherein the CAMLs have one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype,
and wherein the source of the biological sample is one or more of peripheral blood, blood, lymph node, bone marrow, cerebral spinal fluid, tissue, and urine.

3. The method of claim 1, wherein the number of CCR5 clusters is 15 or more, 16 or more, 17 or more, 18 or more, 19 or more, 20 or more, 21 or more, 22 or more, 23 or more, 24 or more, or 25 or more.

4. The method of any one of claims 1-3, wherein said OS and/or PFS is over a period of at least 12 months, or wherein said OS and/or PFS is over a period of at least 24 months.

5. The method of any one of claims 1-4, wherein the size of the biological sample is between 5 and 15 mL.

6. The method of any one of claims 1 to 5, wherein the circulating cells further comprise one or more of CTCs, PDCTCs, apoptotic CTCs, EMTCTCs and CAVEs.

7. The method of any one of claims 1 to 6, wherein the biological sample is antecubital-vein blood, inferior-vena-cava blood, femoral vein blood, portal vein blood, or jugular-vein blood.

8. The method of claim 7, wherein the biological sample is 7.5 ml of antecubital-vein blood, inferior-vena-cava blood, femoral vein blood, portal vein blood, or jugular-vein blood.

9. The method of any one of claims 1-8, wherein the cancer is cancer expressing CCR5 selected from a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

10. The method of any one of claims 1-9, wherein circulating cells are isolated from the biological samples for the determining steps using one or more means selected from the group consisting of size exclusion methodology, immunocapture, red blood cell lysis, white blood cell depletion, FICOLL, electrophoresis, dielectrophoresis, flow cytometry, magnetic levitation, and various microfluidic chips, or a combination thereof.

11. The method of claim 10, wherein circulating cells are isolated from the biological samples using size exclusion methodology that comprises using a microfilter, preferably wherein the microfilter has a pore size ranging from about 5 microns to about 20 microns, optionally wherein the pores of the microfilter have a round, race-track shape, oval, square and rectangular pore shape, or wherein the microfilter has precision pore geometry and uniform pore distribution.

12. The method of claim 10, wherein circulating cells are isolated using a microfluidic chip via physical size-based sorting, slits, channels, hydrodynamic size-based sorting, grouping, trapping, immunocapture, concentrating large cells, or eliminating small cells based on size.

13. The method of any one of claims 1-9 wherein circulating cells are isolated from the biological samples for the determining steps using a low-pressure microfiltration assay.

14. The method of any one of the preceding claims wherein the CCR5 clusters are localized to the nucleus of the cell, or the CCR5 clusters are localized to the cytoplasm of the cell, or localized to the surface of the cell, or some combination thereof; and/or wherein the CCR5 of the circulating cells is in an activated state, or in an inactivated state, or wherein the cells have both activated and inactivated receptors.

15. The method of any one of the preceding claims further comprising using the determination of the number of CCR5 clusters in circulating cells in a biological sample from a subject as a companion or complementary diagnostic in treatment decisions for said subject.

## Patentansprüche

1. Verfahren zum Vorhersagen des Gesamtüberlebens (OS) und progressionsfreien Überlebens (PFS) eines Subjekts, das Krebs aufweist, wobei das Verfahren das Bestimmen der Anzahl von CCR5-Clustern in zirkulierenden Zellen in einer biologischen Probe eines Subjekts, das Krebs aufweist, umfasst,
wobei die zirkulierenden Zellen krebsassoziierte makrophagenähnliche Zellen (CAMLs) umfassen und wobei die CAMLs die folgenden Merkmale aufweisen:
(a) großer, atypischer polyploider Kern mit einer Größe von etwa 14-64 µm oder mehrere Kerne in einer einzelnen Zelle;
(b) Zellgröße von etwa 20-300 µm Größe; und
(c) morphologische Form, ausgewählt aus der Gruppe bestehend aus spindelförmig, kaulquappenförmig, rund, länglich, zweibeinig, mehr als zweibeinig, dünnbeinig und amorph; wobei die CAMLs optional ein oder mehrere der folgenden zusätzlichen Merkmale aufweisen:
(d) CD14-positiver Phänotyp;
(e) CD45-Expression;
(f) EpCAM-Expression;
(g) Vimentin-Expression;
(h) PD-L1-Expression;
(i) CD11C-Marker-Expression;
(j) CD 146-Marker-Expression;
(k) CD202b-Marker-Expression;
(l) CD31-Marker-Expression; und
(m) CK8, 18, 19 epithelialer Phänotyp,
wobei die Quelle der biologischen Probe eines oder mehrere von peripherem Blut, Blut, Lymphknoten, Knochenmark, Zerebrospinalflüssigkeit, Gewebe und Urin ist, und
wobei, wenn mindestens eine CAML-Zelle in der Probe etwa 10 oder mehr CCR5-Cluster aufweist:
(i) das OS und PFS des Subjekts als verkürzt vorhergesagt werden, oder
(ii) das OS und PFS des Subjekts als kürzer vorhergesagt werden als bei eines Subjekts, welches die gleiche Art von Krebs aufweist, bei der keine der Zellen in einer entsprechenden biologischen Probe etwa 10 oder mehr CCR5-Cluster aufweist.

2. Verfahren zum Vorhersagen des OS und PFS eines Subjekts, das Krebs aufweist, wobei das Verfahren umfasst:
(i) Bestimmen der Anzahl von CCR5-Clustern in zirkulierenden Zellen in einer biologischen Probe von einem ersten Subjekt, das Krebs aufweist, und Vergleichen der Ergebnisse mit jenen, die bei einem zweiten Subjekt bestimmt wurden, welches die gleiche Art von Krebs aufweist, wobei das OS und PFS des Subjekts, das eine größere Anzahl von CCRS-Clustern aufweist, als kürzer vorhergesagt werden als das OS und PFS des Subjekts, das weniger CCRS-Cluster aufweist;
(ii) Bestimmen einer mittleren Anzahl von CCR5-Clustern in einer ausgewählten Anzahl von zirkulierenden Zellen in einer biologischen Probe eines ersten Subjekts, das Krebs aufweist, und Vergleichen der Ergebnisse mit jenen, die bei einem zweiten Subjekt bestimmt wurden, welches die gleiche Art von Krebs aufweist, wobei das OS und PFS des Subjekts mit einer größeren mittleren Anzahl von CCRS-Clustern als kürzer vorhergesagt werden als das OS und PFS des Subjekts, das eine kleinere mittlere Anzahl von CCRS-Clustern aufweist; oder
(iii) Bestimmen einer medianen Anzahl von CCR5-Clustern in einer ausgewählten Anzahl von zirkulierenden Zellen in einer biologischen Probe eines ersten Subjekts, das Krebs aufweist, und Vergleichen der Ergebnisse mit jenen, die bei einem zweiten Subjekt bestimmt wurden, welches die gleiche Art von Krebs aufweist, wobei das OS und PFS des Subjekts mit einer größeren medianen Anzahl von CCR5-Clustern als kürzer vorhergesagt werden als das OS und PFS des Subjekts, das eine kleinere mediane Anzahl von CCR5-Clustern aufweist,
wobei die zirkulierenden Zellen krebsassoziierte makrophagenähnliche Zellen (CAMLs) umfassen und wobei die CAMLs die folgenden Merkmale aufweisen:
(a) großer, atypischer polyploider Kern mit einer Größe von etwa 14-64 µm oder mehrere Kerne in einer einzelnen Zelle;
(b) Zellgröße von etwa 20-300 µm Größe; und
(c) morphologische Form, ausgewählt aus der Gruppe bestehend aus spindelförmig, kaulquappenförmig, rund, länglich, zweibeinig, mehr als zweibeinig, dünnbeinig und amorph; wobei die CAMLs optional ein oder mehrere der folgenden zusätzlichen Merkmale aufweisen:
(d) CD14-positiver Phänotyp;
(e) CD45-Expression;
(f) EpCAM-Expression;
(g) Vimentin-Expression;
(h) PD-L1-Expression;
(i) CD11C-Marker-Expression;
(j) CD146-Marker-Expression;
(k) CD202b-Marker-Expression;
(l) CD31-Marker-Expression; und
(m) CK8, 18, 19 epithelialer Phänotyp,
und wobei die Quelle der biologischen Probe eines oder mehrere von peripherem Blut, Blut, Lymphknoten, Knochenmark, Zerebrospinalflüssigkeit, Gewebe und Urin ist.

3. Verfahren nach Anspruch 1, wobei die Anzahl von CCR5-Clustern 15 oder mehr, 16 oder mehr, 17 oder mehr, 18 oder mehr, 19 oder mehr, 20 oder mehr, 21 oder mehr, 22 oder mehr, 23 oder mehr, 24 oder mehr oder 25 oder mehr beträgt.

4. Verfahren nach einem der Ansprüche 1-3, wobei sich das OS und/oder PFS über einen Zeitraum von mindestens 12 Monaten erstreckt, oder wobei sich das OS und/oder PFS über einen Zeitraum von mindestens 24 Monaten erstreckt.

5. Verfahren nach einem der Ansprüche 1-4, wobei die Größe der biologischen Probe zwischen 5 und 15 ml beträgt.

6. Verfahren nach einem der Ansprüche 1 bis 5, wobei die zirkulierenden Zellen weiter eines oder mehrere von CTCs, PDCTCs, apoptotischen CTCs, EMTCTCs und CAVEs umfassen.

7. Verfahren nach einem der Ansprüche 1 bis 6, wobei die biologische Probe Blut aus der Antekubitalvene, Blut aus der unteren Hohlvene, Blut aus der Oberschenkelvene, Blut aus der Pfortader oder Blut aus der Jugularvene ist.

8. Verfahren nach Anspruch 7, wobei die biologische Probe 7,5 ml Blut aus der Antekubitalvene, Blut aus der unteren Hohlvene, Blut aus der Oberschenkelvene, Blut aus der Pfortader oder Blut aus der Jugularvene ist.

9. Verfahren nach einem der Ansprüche 1-8, wobei der Krebs ein Krebs ist, der CCR5 exprimiert, ausgewählt aus einem soliden Tumor, Krebs im Stadium I, Krebs im Stadium II, Krebs im Stadium III, Krebs im Stadium IV, Karzinom, Sarkom, Neuroblastom, Melanom, Epithelzellkrebs, Brustkrebs, Prostatakrebs, Lungenkrebs, Bauchspeicheldrüsenkrebs, Dickdarmkrebs, Leberkrebs, Kopf- und Halskrebs, Nierenkrebs, Eierstockkrebs, Speiseröhrenkrebs, oder einem anderen soliden Tumorkrebs.

10. Verfahren nach einem der Ansprüche 1-9, wobei zirkulierende Zellen aus den biologischen Proben für die Bestimmungsschritte unter Verwendung eines oder mehrerer Mittel isoliert werden, ausgewählt aus der Gruppe bestehend aus Größenausschlussmethodik, Immunocapture, Lyse roter Blutkörperchen, Depletion weißer Blutkörperchen, FICOLL, Elektrophorese, Dielektrophorese, Durchflusszytometrie, magnetischer Levitation und verschiedenen Mikrofluidik-Chips, oder einer Kombination davon.

11. Verfahren nach Anspruch 10, wobei zirkulierende Zellen aus den biologischen Proben unter Verwendung von Größenausschlussmethodik isoliert werden, die das Verwenden eines Mikrofilters umfasst, wobei der Mikrofilter vorzugsweise eine Porengröße im Bereich von etwa 5 Mikrometer bis etwa 20 Mikrometer aufweist, wobei die Poren des Mikrofilters optional eine runde, rennbahnförmige, ovale, quadratische und rechteckige Porenform aufweisen, oder wobei der Mikrofilter Präzisionsporengeometrie und gleichmäßige Porenverteilung aufweist.

12. Verfahren nach Anspruch 10, wobei zirkulierende Zellen unter Verwendung eines Mikrofluidik-Chips durch physikalischem, größenbasierten Sortieren, hydrodynamischem, größenbasierten Sortieren, Gruppieren, Einfangen, Immunocapture, Konzentrieren großer Zellen oder Eliminieren kleiner Zellen, basierend auf Größe isoliert werden.

13. Verfahren nach einem der Ansprüche 1-9, wobei zirkulierende Zellen aus den biologischen Proben für die Bestimmungsschritte unter Verwendung eines Niederdruck-Mikrofiltrationsassays isoliert werden.

14. Verfahren nach einem der vorstehenden Ansprüche, wobei die CCRS-Cluster im Kern der Zelle lokalisiert sind, oder die CCR5-Cluster im Zytoplasma der Zelle lokalisiert sind, oder auf der Oberfläche der Zelle lokalisiert sind, oder eine Kombination davon; und/oder wobei sich der CCR5 der zirkulierenden Zellen in einem aktivierten Zustand oder in einem inaktivierten Zustand befindet oder wobei die Zellen beide, aktivierte und deaktivierte Rezeptoren aufweisen.

15. Verfahren nach einem der vorstehenden Ansprüche, das weiter das Verwenden der Bestimmung der Anzahl von CCR5-Clustern in zirkulierenden Zellen in einer biologischen Probe eines Subjekts als begleitende oder ergänzende Diagnose bei Behandlungsentscheidungen für das Subjekt umfasst.

## Revendications

1. Procédé d'estimation de la survie globale (SG) et de la survie sans progression (SSP) d'un sujet atteint d'un cancer, ledit procédé comprenant la détermination du nombre de groupes CCR5 dans des cellules circulantes dans un échantillon biologique provenant d'un sujet atteint d'un cancer,
dans lequel les cellules circulantes comprennent des cellules de type macrophage associées au cancer (CAML) et dans lequel les CAML présentent les caractéristiques suivantes :
(a) grand noyau polyploïde atypique d'une taille d'environ 14-64 µm, ou plusieurs noyaux dans une seule cellule ;
(b) taille de cellule d'environ 20-300 µm ; et
(c) forme morphologique sélectionnée dans le groupe consistant en fuseau, têtard, ronde, oblongue, à deux pattes, à plus de deux pattes, à pattes fines et amorphes ; éventuellement, dans lequel les CAML présentent une ou plusieurs des caractéristiques supplémentaires suivantes :
(d) phénotype CD14 positif ;
(e) expression de CD45 ;
(f) expression d'EpCAM ;
(g) expression de vimentine ;
(h) expression de PD-L1 ;
(i) expression du marqueur CD11C ;
(j) expression du marqueur CD146 ;
(k) expression du marqueur CD202b ;
(l) expression du marqueur CD31 ; et
(m) phénotype épithélial CK8, 18, 19,
dans lequel la source de l'échantillon biologique est un ou plusieurs éléments parmi le sang périphérique, le sang, les ganglions lymphatiques, la moelle osseuse, le liquide céphalo-rachidien, les tissus et l'urine, et
dans lequel, quand au moins une cellule CAML dans ledit échantillon présente environ 10 groupes CCR5 ou plus
(i) il est estimé que la SG et la SSP du sujet sont raccourcies, ou
(ii) il est estimé que la SG et la SSP du sujet sont plus courtes que celles d'un sujet atteint du même type de cancer pour lequel aucune des cellules d'un échantillon biologique correspondant ne présente environ 10 groupes CCR5 ou plus.

2. Procédé d'estimation de la SG et la SSP d'un sujet atteint d'un cancer, ledit procédé comprenant :
(i) la détermination du nombre de groupes CCR5 dans les cellules circulantes dans un échantillon biologique provenant d'un premier sujet atteint d'un cancer, et la comparaison des résultats à ceux déterminés à partir d'un second sujet atteint du même type de cancer, dans lequel il est estimé que la SG et la SSP du sujet présentant un plus grand nombre de groupes CCR5 sont plus courtes que la SG et la SSP du sujet présentant moins de groupes CCR5 ;
(ii) la détermination d'un nombre moyen de groupes CCR5 dans un nombre sélectionné de cellules circulantes dans un échantillon biologique provenant d'un premier sujet atteint d'un cancer, et la comparaison des résultats à ceux déterminés à partir d'un second sujet atteint du même type de cancer, dans lequel il est estimé que la SG et la SSP du sujet avec un nombre moyen plus élevé de groupes CCR5 sont plus courtes que la SG et la SSP du sujet présentant un nombre moyen de groupes CCR5 inférieur ; ou
(iii) la détermination d'un nombre médian de groupes CCR5 dans un nombre sélectionné de cellules circulantes dans un échantillon biologique provenant d'un premier sujet atteint d'un cancer, et la comparaison des résultats à ceux déterminés à partir d'un second sujet atteint du même type de cancer, dans lequel il est estimé que la SG et la SSP du sujet présentant un nombre médian plus élevé de groupes CCR5 sont plus courtes que la SG et la SSP du sujet présentant un nombre médian de groupes CCR5 inférieur,
dans lequel les cellules circulantes comprennent des cellules de type macrophage associées au cancer (CAML) et dans lequel les CAML présentent les caractéristiques suivantes :
(a) grand noyau polyploïde atypique d'une taille d'environ 14-64 µm, ou plusieurs noyaux dans une seule cellule ;
(b) taille de cellule d'environ 20-300 µm ; et
(c) forme morphologique sélectionnée dans le groupe consistant en fuseau, têtard, ronde, oblongue, à deux pattes, à plus de deux pattes, à pattes fines et amorphes ; éventuellement, dans lequel les CAML présentent une ou plusieurs des caractéristiques supplémentaires suivantes :
(d) phénotype CD14 positif ;
(e) expression de CD45 ;
(f) expression d'EpCAM ;
(g) expression de vimentine ;
(h) expression de PD-L1 ;
(i) expression du marqueur CD11C ;
(j) expression du marqueur CD146 ;
(k) expression du marqueur CD202b ;
(l) expression du marqueur CD31 ; et
(m) phénotype épithélial CK8, 18, 19,
et dans lequel la source de l'échantillon biologique est un ou plusieurs éléments parmi le sang périphérique, le sang, les ganglions lymphatiques, la moelle osseuse, le liquide céphalo-rachidien, les tissus et l'urine.

3. Procédé selon la revendication 1, dans lequel le nombre de groupes CCR5 est de 15 ou plus, 16 ou plus, 17 ou plus, 18 ou plus, 19 ou plus, 20 ou plus, 21 ou plus, 22 ou plus, 23 ou plus, 24 ou plus, ou 25 ou plus.

4. Procédé selon l'une quelconque des revendications 1-3, dans lequel ladite SG et/ou ladite SSP s'étend(ent) sur une période d'au moins 12 mois, ou dans lequel ladite SG et/ou ladite SSP s'étend(ent) sur une période d'au moins 24 mois.

5. Procédé selon l'une quelconque des revendications 1-4, dans lequel la taille de l'échantillon biologique est comprise entre 5 et 15 ml.

6. Procédé selon l'une quelconque des revendications 1 à 5, dans lequel les cellules circulantes comprennent en outre un ou plusieurs parmi les CTC, PDCTC, CTC apoptotiques, EMTCTC et CAVE.

7. Procédé selon l'une quelconque des revendications 1 à 6, dans lequel l'échantillon biologique est du sang de la veine antécubitale, du sang de la veine cave inférieure, du sang de la veine fémorale, du sang de la veine porte ou du sang de la veine jugulaire.

8. Procédé selon la revendication 7, dans lequel l'échantillon biologique est constitué de 7,5 ml de sang de la veine antécubitale, de sang de la veine cave inférieure, de sang de la veine fémorale, de sang de la veine porte ou de sang de veine jugulaire.

9. Procédé selon l'une quelconque des revendications 1-8, dans lequel le cancer est un cancer exprimant CCRS sélectionné parmi une tumeur solide, un cancer de stade I, un cancer de stade II, un cancer de stade III, un cancer de stade IV, un carcinome, un sarcome, un neuroblastome, un mélanome, un cancer de cellule épithéliale, un cancer du sein, un cancer de la prostate, un cancer du poumon, un cancer du pancréas, un cancer colorectal, un cancer du foie, un cancer de la tête et du cou, un cancer du rein, un cancer des ovaires, un cancer de l'œsophage ou un autre cancer à tumeur solide.

10. Procédé selon l'une quelconque des revendications 1-9, dans lequel les cellules circulantes sont isolées des échantillons biologiques pour les étapes de détermination en utilisant un ou plusieurs moyens sélectionnés dans le groupe consistant en la méthodologie d'exclusion de taille, l'immunocapture, la lyse des globules rouges, la déplétion des globules blancs, le FICOLL, l'électrophorèse, la diélectrophorèse, la cytométrie en flux, la lévitation magnétique et diverses puces microfluidiques, ou une combinaison de celles-ci.

11. Procédé selon la revendication 10, dans lequel les cellules circulantes sont isolées des échantillons biologiques en utilisant une méthodologie d'exclusion de taille qui comprend l'utilisation d'un microfiltre, de préférence dans lequel le microfiltre présente une taille de pores allant d'environ 5 micromètres à environ 20 micromètres, éventuellement dans lequel les pores du microfiltre présentent une forme ronde, une forme de circuit, une forme de pores ovale, carrée et rectangulaire, ou dans lequel le microfiltre présente une géométrie de pores précise et une distribution uniforme des pores.

12. Procédé selon la revendication 10, dans lequel les cellules circulantes sont isolées en utilisant une puce microfluidique via un tri basé sur la taille physique, des fentes, des canaux, un tri hydrodynamique basé sur la taille, un regroupement, un piégeage, une immunocapture, une concentration de grandes cellules ou une élimination de petites cellules sur la base de la taille.

13. Procédé selon l'une quelconque des revendications 1-9, dans lequel les cellules circulantes sont isolées des échantillons biologiques pour les étapes de détermination en utilisant un test de microfiltration à basse pression.

14. Procédé selon une quelconque revendication précédente, dans lequel les groupes CCR5 sont localisés dans le noyau de la cellule, ou les groupes CCR5 sont localisés dans le cytoplasme de la cellule, ou localisés à la surface de la cellule, ou une combinaison de ceux-ci ; et/ou dans lequel le CCR5 des cellules circulantes est dans un état activé ou dans un état inactivé, ou dans lequel les cellules présentent à la fois des récepteurs activés et inactivés.

15. Procédé selon l'une quelconque des revendications précédentes, comprenant en outre l'utilisation de la détermination du nombre de groupes CCRS dans les cellules circulantes dans un échantillon biologique provenant d'un sujet comme diagnostic compagnon ou complémentaire lors des décisions de traitement pour ledit sujet.
